# EUROPEAN PATENT APPLICATION

(11) **EP 4 245 812 A1**
(43) Date of publication of application: **20.09.2023**
(21) Application number: 21891237.6
(22) Date of filing: 12.11.2021
(51) Int. Cl.: C09B 23/10, C07D 417/06, C07D 513/04

(54) **LEUKOCYTE CLASSIFICATION REAGENT, ERYTHROCYTE ANALYSIS REAGENT, KITS, AND ANALYSIS METHOD**

(30) Priority: 13.11.2020 WO PCT/CN2020/128839
(71) Applicant: Dalian University of Technology, Dalian, Liaoning 116024 (CN); Shenzhen Mindray Bio-Medical Electronics Co., Ltd, Shenzhen, Guangdong 518057 (CN)
(72) Inventor: FAN, Jiangli, Dalian, Liaoning 116024 (CN); CHEN, Gengwen, Shenzhen, Guangdong 518057 (CN); XIA, Tianping, Dalian, Liaoning 116024 (CN); YE, Yi, Shenzhen, Guangdong 518057 (CN); ZHANG, Ziqian, Shenzhen, Guangdong 518057 (CN); YAO, Qichao, Dalian, Liaoning 116024 (CN)
(74) Representative: KIPA AB
(86) International application number: PCT/CN2021/130478
(87) International publication number: WO 2022/100717

(57) **Abstract**

Disclosed are a leukocyte classification reagent and kit, a classification method, an erythrocyte classification reagent and kit, and an analysis method. Both the leukocyte classification reagent and the erythrocyte classification reagent comprise a fluorescent dye represented by the general general formula F. The dye can stain the nucleic acids of leukocytes and erythrocytes, therefore, during blood analysis, the classification and/or counting of leukocytes in a sample can be accurately realized, and the classification and/or counting of erythrocytes in a sample can be accurately realized, in particular the identification and/or counting of RETs. In addition, the types of dyes used in blood cell analysis may also be simplified.

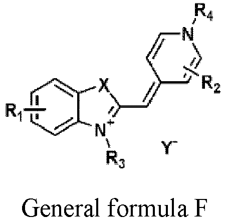

## Description

### TECHNICAL FIELD

The disclosure relates to an erythrocyte classification reagent, a leukocyte analysis reagent, and methods of use thereof, and in particular to a reagent for automatically classifying various leukocyte subsets in blood by an analyzer, a reagent for identifying erythrocytes and types thereof in blood by an analyzer, and the methods.

### BACKGROUND

Blood cells are divided into three types of cells: erythrocytes (red blood cell, RBC), leukocytes (white blood cell, WBC), and platelets (PLT). The white blood cell count and subsets, red blood cell count and platelet count are often required to be detected during blood routine test.

Generally, leukocytes in normal peripheral blood can be classified into five subsets, i.e., lymphocytes, monocytes, neutrophils, eosinophils, and basophils. Analysis of leukocyte populations in a blood sample can provide much useful information for clinical diagnosis of numerous diseases. For example, the onset of a disease can alter the proportion and number of various leukocyte subsets in blood. Additionally, it can also be accompanied by abnormal leukocytes such as abnormal lymphocytes and immature granulocytes. Therefore, the classification and determination of various types of normal and abnormal leukocytes can provide information about a disease, including its stages of latency, onset, and development.

Erythrocytes in normal peripheral blood include mature erythrocytes and a small number of reticulocytes (RET). Reticulocytes (RET) are transitional cells from orthochromatic erythrocytes to mature erythrocytes. They are slightly larger than mature erythrocytes. Reticulocytes are at a stage where nucleated red blood cells have just lost their nuclei, and still immature red blood cells. They contain residual basophilic substances such as ribosomes, ribonucleic acids in their cytoplasm. After vital staining with brilliant cresyl blue or new methylene blue, blue or blue-green branched or even reticular structures can be observed in their cytoplasm, which is why they are referred to as reticulocytes. Current researches suggest that reticulocytes, as an important stage in the maturation of red blood cells, should also be paid due attention.

Fluorescence analysis methods for current analyzers use different dyes to analyze erythrocytes and leukocytes, which makes the design of the apparatus and liquid path more complex. Therefore, there is a need for a new dye that can be used to classify and count erythrocytes and leukocytes, so as to simplify the structure of hematology analyzers.

### SUMMARY

In the Summary, a series of concepts in a simplified form are introduced and will be further described in detail in the Detailed Description. The summary of the disclosure is not intended to define the key features and essential technical features of the claimed technical solutions, nor is it intended to determine the protection scope of the claimed technical solutions.

In order to at least partially solve the above-mentioned problem, a first aspect of the disclosure provides a leukocyte classification reagent including a fluorescent dye represented by general formula F: 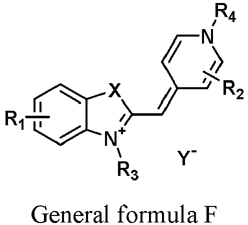
in which X is selected from a group consisting of C(CH₃)₂, O, S and Se;
R₁ and R₂ are each independently selected from a group consisting of H, Ci-Cis alkyl, phenyl, OR₆ and halogen;
R₃ and R₄ are each independently selected from a group consisting of Ci-Cis alkyl, Ci-Cis-carboxyl, Ci-Cis-hydroxyl, C₁-C₁₈NR₅R₆, benzyl and substituted benzyl which is substituted with a substituent selected from a group consisting of C₁-C₁₈ alkyl, CN, COOH, NH₂, NO₂, OH, SH, C₁-C₆ alkoxy, C₁-C₆ alkylamino, C₁-C₆ amide, halogen and C₁-C₆ haloalkyl;
R₅ and R₆ are each independently selected from a group consisting of H and Ci-Cis alkyl; and
Y⁻ is an anion.

The fluorescent dye represented by general formula F in the disclosure is a cyanine compound, which has good viable cell permeability, and can penetrate cells without damaging the cell membrane, to stain the nucleic acids. During blood analysis, the fluorescent dye stains leukocytes, enabling the classification and/or counting of these leukocyte cells. In one embodiment, the excitation light of the dye is blue-green light with a smaller wavelength, achieving the identification of small particles and thereby enhancing the ability to detect the small particles.

A second aspect of the disclosure provides a leukocyte analysis kit including the leukocyte classification reagent as described in the first aspect. The leukocyte analysis kit of the disclosure allows classify and/or count leukocytes during the blood analysis; in which the leukocyte analysis kit enable classify the leukocytes into lymphocytes, monocytes, neutrophils, and eosinophils and count the classified cells.

A third aspect of the disclosure provides a method for analyzing leukocytes, which includes mixing a blood sample, a fluorescent dye represented by general formula F and an erythrolysis agent to form a mixture, in which the general formula F is shown below: 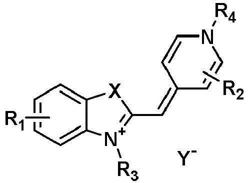
in which X, R₁, R₂, R₃, R₄ and Y⁻ are as defined for the leukocyte classification reagent in the first aspect;
detecting at least one scattered light characteristic and at least one fluorescence characteristic of the sample to be tested by irradiating particles in the sample to be tested with light; and
classifying leukocytes, or classifying and counting leukocytes based on the scattered light characteristic and the fluorescence characteristic.

A fourth aspect of the disclosure provides an erythrocyte classification reagent including a fluorescent dye represented by general formula F, in which the general formula F is shown below: 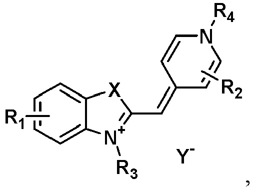 in which X, R₁, R₂, R₃, R₄ and Y⁻ are as defined for the leukocyte classification reagent in the first aspect. The erythrocyte classification reagent stains erythrocytes in the blood analysis to obtain their parameters.

A fifth aspect of the disclosure provides an erythrocyte analysis kit including the erythrocyte classification reagent as described in the fourth aspect. The leukocyte analysis kit of the disclosure allows classify and/or count erythrocytes during the blood analysis. The classification of the erythrocytes includes classifying the erythrocytes into mature erythrocytes and/or reticulocytes; and the counting of the erythrocytes includes counting the mature erythrocytes and/or the reticulocytes.

A sixth aspect of the disclosure provides an erythrocyte analysis method, which includes: mixing a blood sample, a fluorescent dye represented by general formula F and a spheroidizing reagent to form a mixture, in which the general formula F is shown below: 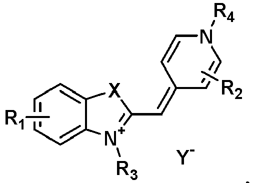
in which X, R₁, R₂, R₃, R₄ and Y⁻ are as defined for the leukocyte classification reagent in the first aspect;
detecting at least one scattered light characteristic and at least one fluorescence characteristic of the mixture; and
classifying erythrocytes or classifying and counting erythrocytes based on the scattered light characteristic and the fluorescence characteristic.

The leukocyte classification reagent and the erythrocyte classification reagent in the disclosure both include the fluorescent dye represented by general formula F, and the dye can stain the nucleic acids of both leukocytes and erythrocytes. Therefore, it is possible to accurately classify and/or count both the leukocytes and the erythrocytes, especially RETs, in a sample during the blood analysis. In addition, the types of dyes used in the blood cell analysis can also be reduced.

### BRIEF DESCRIPTION OF THE DRAWINGS

The accompanying drawings of the disclosure are included herein as a part of the disclosure for the purpose of understanding the disclosure. The drawings illustrate the embodiments of the disclosure and the description thereof to better explain the principles of the disclosure, in which:
FIG. 1 shows a side scattered light-fluorescence scatterplot using the compound represented by chemical formula I as a fluorescent dye for analyzing leukocytes;
FIG. 2 shows a fluorescence-forward scattered light scatterplot using the compound represented by chemical formula I as a fluorescent dye for detecting erythrocytes and platelets;
FIG. 3 shows a side scattered light-fluorescence scatterplot using the compound represented by chemical formula II as a fluorescent dye for analyzing leukocytes;
FIG. 4 shows a fluorescence-forward scattered light scatterplot using the compound represented by chemical formula II as a fluorescent dye for detecting erythrocytes and platelets;
FIG. 5 shows a side scattered light-fluorescence scatter diagram using the compound represented by chemical formula III as a fluorescent dye for analyzing leukocytes;
FIG. 6 shows a fluorescence-forward scattered light scatterplot using the compound represented by chemical formula III as a fluorescent dye for detecting erythrocytes and platelets;
FIG. 7 shows a side scattered light-fluorescence scatterplot using the compound represented by chemical formula IV as a fluorescent dye for analyzing leukocytes;
FIG. 8 shows a fluorescence-forward scattered light scatterplot using the compound represented by chemical formula IV as a fluorescent dye for detecting erythrocytes and platelets;
FIG. 9 shows a side scattered light-fluorescence scatterplot using the compound represented by chemical formula V as a fluorescent dye for analyzing leukocytes;
FIG. 10 shows a fluorescence-forward scattered light scatterplot using the compound represented by chemical formula V as a fluorescent dye for detecting erythrocytes and platelets;
FIG. 11 shows a side scattered light-fluorescence scatterplot using the compound represented by chemical formula VI as a fluorescent dye for analyzing leukocytes;
FIG. 12 shows a fluorescence-forward scattered light scatterplot using the compound represented by chemical formula VI as a fluorescent dye for detecting erythrocytes and platelets;
FIG. 13 shows a side scattered light-fluorescence scatterplot using the compound represented by chemical formula VII as a fluorescent dye for analyzing leukocytes;
FIG. 14 shows a fluorescence-forward scattered light scatterplot using the compound represented by chemical formula VII as a fluorescent dye for detecting erythrocytes and platelets;
FIG. 15 shows a side scattered light-fluorescence scatterplot using the compound represented by chemical formula VIII as a fluorescent dye for analyzing leukocytes;
FIG. 16 shows a fluorescence-forward scattered light scatterplot using the compound represented by chemical formula VIII as a fluorescent dye for detecting erythrocytes and platelets;
FIG. 17 shows a side scattered light-fluorescence scatterplot using the compound represented by chemical formula IX as a fluorescent dye for analyzing leukocytes;
FIG. 18 shows a fluorescence-forward scattered light scatterplot using the compound represented by chemical formula IX as a fluorescent dye for detecting erythrocytes and platelets;
FIG. 19 shows a side scattered light-fluorescence scatterplot using the compound represented by chemical formula X as a fluorescent dye for analyzing leukocytes;
FIG. 20 shows a fluorescence-forward scattered light scatterplot using the compound represented by chemical formula X as a fluorescent dye for detecting erythrocytes and platelets;
FIG. 21 shows a side scattered light-fluorescence scatterplot using the compound represented by chemical formula XI as a fluorescent dye for analyzing leukocytes;
FIG. 22 shows a fluorescence-forward scattered light scatterplot using the compound represented by chemical formula XI as a fluorescent dye for detecting erythrocytes and platelets;
FIG. 23 shows a side scattered light-fluorescence scatterplot using the compound represented by chemical formula XII as a fluorescent dye for analyzing leukocytes;
FIG. 24 shows a fluorescence-forward scattered light scatterplot using the compound represented by chemical formula XII as a fluorescent dye for detecting erythrocytes and platelets;
FIG. 25 shows a side scattered light-fluorescence scatterplot using the compound represented by chemical formula XIII as a fluorescent dye for analyzing leukocytes;
FIG. 26 shows a fluorescence-forward scattered light scatterplot using the compound represented by chemical formula XIII as a fluorescent dye for detecting erythrocytes and platelets;

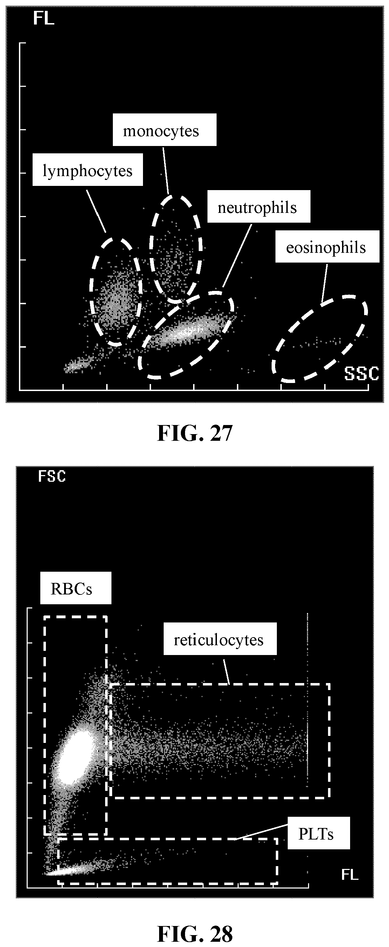

FIG. 27 shows a side scattered light-fluorescence scatterplot diagram using the compound represented by chemical formula XVIII as a fluorescent dye for analyzing leukocytes; and
FIG. 28 shows a fluorescence-forward scattered light scatterplot using the compound represented by chemical formula XVIII as a fluorescent dye for detecting erythrocytes and platelets.

### DETAILED DESCRIPTION

In the following description, numerous specific details are given to provide a more thorough understanding of the disclosure. However, it is apparent to those skilled in the art that the disclosure can be implemented without one or more of these details. In other examples, some well-known technical features in the art are not described in order to avoid obscuring the disclosure.

For a thorough understanding, the disclosure will be described in more detail in the following description. Apparently, the execution of the embodiments of the disclosure is not limited to special details that are familiar to those skilled in the art. The preferred embodiments of the disclosure are described in detail as follows. However, the disclosure can also have other embodiments in addition to these detailed descriptions.

It should be noted that the terms used herein are only for the purpose of describing specific embodiments and are not intended to limit the exemplary embodiments according to the disclosure. As used herein, the singular form is also intended to include the plural form, unless the context explicitly indicates otherwise. In addition, it is to be further understood that the terms "comprise" and/or "include" used in the specification specify the presence of the features, integers, steps, operations, elements and/or components, but do not exclude the presence or addition of one or more of other features, integers, steps, operations, elements, components and/or their combinations.

The term "blood" or "blood sample" used herein refers to a body fluid sample containing blood cells, such as, peripheral blood and bone marrow fluid, unless otherwise specified.

In particular, as used throughout the disclosure, the term "alkyl" can be understood in the broadest sense as any linear, branched or cyclic alkyl substituent. As used herein, the term "C₁₋₁₈ alkyl" generally refers to a saturated hydrocarbon group having 1 to 18 carbon atoms in its configuration, and the C₁₋₁₈ alkyl includes, but is not limited to, C₁₋₁₂ alkyl, C₁₋₆ alkyl, or the like. The term "alkyl" generally refers to un-substituted alkyl, unless described as "substituted alkyl". By way of example, the term "alkyl" includes methyl (Me), ethyl (Et), n-propyl (nPr), isopropyl (iPr), cyclopropyl, n-butyl (nBu), isobutyl (iBu), sec-butyl (sBu), tert-butyl (tBu), cyclobutyl, 2-methylbutyl, n-pentyl, sec-pentyl, tert-pentyl, 2-pentyl, neopentyl, cyclopentyl, n-hexyl, sec-hexyl, tert-hexyl, 2-hexyl, 3-hexyl, neohexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo [2,2,2]octyl, 2-bicyclo [2,2,2]-octyl, 2-(2,6-dimethyl)octyl, 3-(3,7-dimethyl)octyl, adamantyl, 2,2,2-trifluoroethyl, 1,1-dimethyl-n-hex-1-yl, 1,1-dimethyl-n-hept-1-yl, 1,1-dimethyl-n-oct-1-yl, 1,1-dimethyl-n-decan-1-yl, 1,1-dimethyl-n-dodecan-1-yl, 1,1-dimethyl-n-tetradecan-1-yl, 1,1-dimethyl-n-hexadecan-1-yl, 1,1-dimethyl-n-octadecan-1-yl, 1,1-diethyl-n-hex-1-yl, 1,1-diethyl-n-hept-1-yl, 1,1-diethyl-n-oct-1-yl, 1,1-diethyl-n-decan-1-yl, 1,1-diethyl-n-dodecan-1-yl, 1,1-diethyl-n-tetradecan-1-yl, 1,1-diethyl-n-hexadecan-1-yl, 1, 1-diethyl-n-octadecan-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl, and 1-(n-decyl)-cyclohex-1-yl, and the like.

As used above and herein, the term "carboxyl" includes any linear, branched or cyclic carboxyl substituent. As used herein, the term "Ci-is carboxyl" generally refers to a carboxyl substituent having 1 to 18 carbon atoms in its configuration. C₁₋₁₈ carboxyl includes, but is not limited to, C₁₋₁₂ carboxyl, C₁₋₆ carboxyl, and the like. In particular, the term "carboxyl" refers to a group having a carboxyl group and connected to an alkyl group as defined above. By way of example, the term "carboxyl" includes methylcarboxyl, ethyl carboxyl, propylcarboxyl, butylcarboxyl, pentylcarboxyl, hexylcarboxyl, heptylcarboxyl, and octylcarboxyl, as well as isomers thereof, etc.

As used above and herein, the term "hydroxyl" includes any linear, branched or cyclic hydroxyl substituent. As used herein, the term "Ci-is hydroxyl" generally refers to a hydroxyl substituent having 1 to 18 carbon atoms in its configuration.Ci-is hydroxyl includes, but is not limited to, C₁₋₁₂ hydroxyl, C₁₋₆ hydroxyl, and the like. In particular, the term "hydroxyl" refers to a group having a hydroxyl group and connected to an alkyl group as defined above. By way of example, the term "hydroxyl" includes hydroxymethyl, hydroxyethyl, hydroxypropyl, hydroxybutyl, hydroxypentyl, hydroxyhexyl, hydroxyheptyl, and hydroxyoctyl, as well as isomers thereof, etc.

As used above and herein, the term "alkoxy" includes any linear, branched or cyclic alkoxy substituent. In particular, the term "alkoxy" refers to an alkoxy group connected to an alkyl group as defined above. By way of example, the term "alkoxy" includes methoxy, ethoxy, n-propoxy, isopropoxy, n-butoxy, isobutoxy, sec-butoxy, tert-butoxy, 2-methylbutoxy, and the like.

As used above and herein, the terms "halogen" and "halo" can be understood in the broadest sense as preferably fluorine, chlorine, bromine, or iodine.

As used herein, the wavelength of the blue-green light is in the range of about 400-560 nm, unless more specifically defined in particular contexts.

### Fluorescent Dye

The fluorescent dye of the disclosure is a cyanine compound, which has good viable cell permeability, and can enter cells without damaging the cell membrane, to specifically stain the nucleic acids (RNA and DNA). The dye is a fluorescent dye that is excited by blue-green light. The dye can be excited by blue or green laser emitted from a device that provides laser in a blue or green spectral region, such as a blue or green semiconductor laser device. Therefore, the reagent of the disclosure can be used in hematology analyzers or flow cytometers that utilize an inexpensive device, such as a semiconductor laser device, as a light source.

In one embodiment, the fluorescent dye has the structure represented by general formula F: 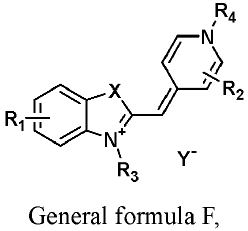
in which X is selected from the group consisting of C(CH₃)₂, O, S and Se;
R₁ and R₂ are each independently selected from the group consisting of H, C₁-C₁₈ alkyl, phenyl, OR₆ and halogen;
R₃ and R₄ are each independently selected from the group consisting of Ci-Cis alkyl, Ci-Cis-carboxyl, Ci-Cis-hydroxyl, C₁-C₁₈NR₅R₆, benzyl and substituted benzyl which is substituted with a substituent selected from the group consisting of C₁-C₁₈ alkyl, CN, COOH, NH₂, NO₂, OH, SH, C₁-C₆ alkoxy, C₁-C₆ alkylamino, C₁-C₆ amide, halogen and C₁-C₆ haloalkyl;
R₅ and R₆ are each independently selected from the group consisting of H and Ci-Cis alkyl; and
Y⁻ is an anion.

In one embodiment, X in the general formula F is selected from the group consisting of C(CH₃)₂ and S.

In one embodiment, R₁ and R₂ in the general formula F are each independently selected from the group consisting of H, C₁-C₁₂ alkyl, phenyl, OR₆ and halogen.

In one embodiment, R₁ and R₂ in the general formula F are each independently selected from the group consisting of H, C₁-C₆ alkyl, phenyl, OR₆ and halogen.

In one embodiment, R₁ in the general formula F is selected from the group consisting of H, C₁-C₆ alkyl, phenyl and halogen.

In one embodiment, R₂ in the general formula F is H.

In one embodiment, R₃ and R₄ in the general formula F are each independently selected from the group consisting of C₁-C₁₂ alkyl, C₁-C₁₂-carboxyl, C₁-C₁₂-hydroxyl, C₁-C₁₂NR₅R₆, benzyl and substituted benzyl which is substituted with a substituent selected from the group consisting of C₁-C₁₂ alkyl, CN, COOH, NH₂, NO₂, OH, SH, C₁-C₆ alkoxy, C₁-C₆ alkylamino, C₁-C₆ amide, halogen and C₁-C₆ haloalkyl.

In one embodiment, R₃ and R₄ in the general formula F are each independently selected from the group consisting of C₁-C₆ alkyl, C₁-C₆-carboxyl, C₁-C₆-hydroxyl, C₁-C₆NR₅R₆, benzyl and substituted benzyl which is substituted with a substituent selected from the group consisting of C₁-C₆ alkyl, CN, COOH, NH₂, NO₂, OH, SH, C₁-C₆ alkoxy, C₁-C₆ alkylamino, C₁-C₆ amide, halogen and C₁-C₆ haloalkyl.

In one embodiment, R₃ in the general formula F is selected from the group consisting of C₁-C₆ alkyl, C₁-C₆-hydroxyl, C₁-C₆-carboxyl, C₁-C₆NR₅R₆ and benzyl.

In one embodiment, R₄ in the general formula F is selected from the group consisting of C₁-C₆ alkyl, C₁-C₆-hydroxyl, C₁-C₆-carboxyl and benzyl.

In one embodiment, R₅ and R₆ in the general formula F are each independently selected from the group consisting of H and C₁-C₁₂ alkyl.

In one embodiment, R₅ and R₆ in the general formula F are each independently selected from the group consisting of H and C₁-C₆ alkyl.

In one embodiment, R₅ and R₆ in the general formula F are each independently C₁-C₆ alkyl.

In one embodiment, Y⁻ in the general formula F is selected from the group consisting of an anion of halogen, ClO₄⁻, PF₆⁻, BF₄⁻, CH₃COO⁻ and OTs⁻.

In one embodiment, the fluorescent dye is represented by chemical formula I, chemical formula II, chemical formula III, chemical formula IV, chemical formula V, chemical formula VI, chemical formula VII, chemical formula VIII, chemical formula IX, chemical formula X, chemical formula XI, chemical formula XII, chemical formula XIII, chemical formula XIV, chemical formula XV, chemical formula XVI, chemical formula XVII or chemical formula XVIII:

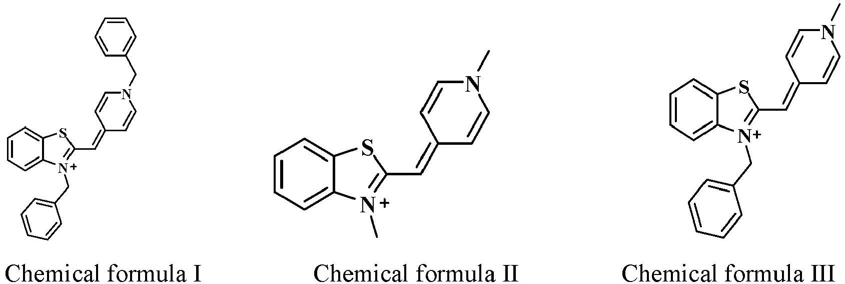

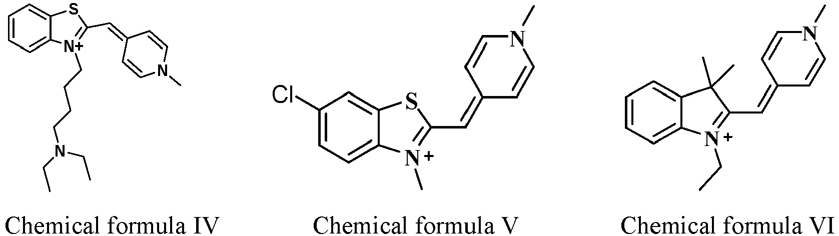

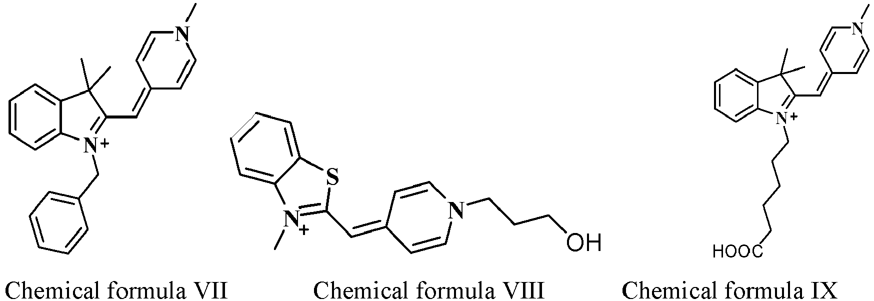

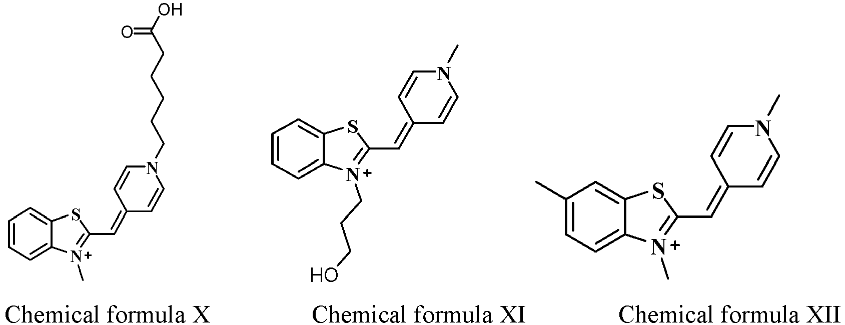

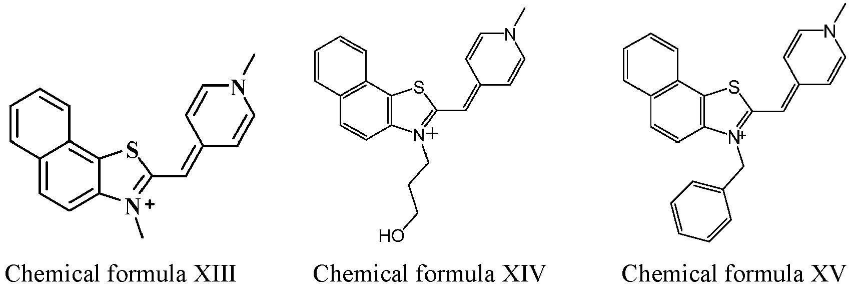

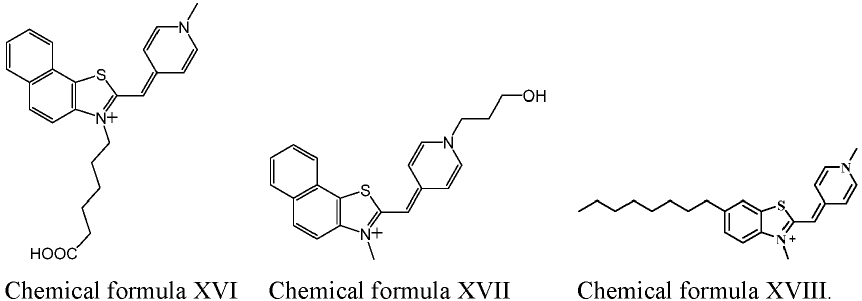

The fluorescent dye of the disclosure can be dissolved in glycerol, glycol and ethylene glycol, to be preserved as a stock solution, or can be dissolved in other non-aqueous solvents. In the disclosure, ethylene glycol is preferably used as a solvent, and optionally 5-10% of methanol is contained, serving as a diluent ingredient. In addition, according to the disclosure, a non-ionic surfactant is optionally adopted as a dispersant, to prevent the fluorescent dye from aggregating, such that the fluorescent dye maintains certain solubility and is prevented from aggregation. Such a non-ionic surfactant is, for example, polyoxyethylene (POE), polyoxypropylene (POP), polyoxyethylene -polyoxypropylene (POE-POP) and a Brij series non-ionic surfactants. In the disclosure, Brij 35, Brij 56,or the like preferably serves as a dispersant for the fluorescent dye, and the concentration thereof is 0.02-2%.

Alternatively, the fluorescent dye of the disclosure can be formulated with an erythrolysis agent or a spheroidizing reagent to form a single reagent.

### Erythrolysis agent

The erythrolysis agent may include a cationic surfactant, a non-ionic surfactant, an anionic surfactant or any combination thereof; and a buffer that maintains the pH of the tested system in the range of 5-11.

The adopted cationic surfactant, non-ionic surfactant, anionic surfactant, or any combination thereof may serve as a reagent for lysing erythrocytes. The surfactant allows lyse the erythrocytes in a blood sample, and appropriately disrupt the membrane structures of leukocytes of various subsets, such that the leukocyte of the subsets in the blood sample contract to an appropriate size and facilitate aggregation of the internal structures of the leukocytes at different levels, thereby resulting in different scattered light characteristics. The cationic surfactant can be octyltrimethylammonium bromide (OTAB), decyltrimethyl ammonium bromide (DTAB), lauryltrimethyl ammonium chloride (LTAC), tetradecyltrimethyl ammonium bromide (CTAB), tetradecyltrimethyl ammonium chloride (CTAC), or the like, preferably LTAC. The cationic surfactant is used at a concentration of 300-800 mg/L. The non-ionic surfactant may be a polyoxyethylene type non-ionic surfactant, for example, long chain fatty alcohol polyoxyethylene, alkylphenol ethoxylates, polyoxyethylene fatty acid, fatty amine polyoxyethylene ether, or the like, preferably polyoxyethylene-2,3-lauryl ether (Brij35). The non-ionic surfactant is used at a concentration of 1-5 g/L. The anionic surfactant is selected from dodecylbenzene sulfonic acid, sodium dodecyl sulfate, sodium ethoxylated fatty acid methyl ester sulfonate, sodium sec-alkyl sulfonate and alcohol ether carboxylate. The anionic surfactant is used at a concentration of 0.1-2 g/L.

The reagent of the disclosure may contain a buffer that maintains the pH and appropriately dilutes the blood sample. The buffer maintains the pH within a constant range, thereby stabilizing the staining effects of various leukocyte subsets. The buffer is used at the concentration of 0.01-200 mM. The type of buffer is not specifically limited, as long as it is suitable for the purposes of the disclosure, such as a carboxylate, a phosphate, a citrate, Tris-HCl, MOPS, as well as other organic buffers at an appropriate concentration. The suitable pH of the reagent in the disclosure varies depending on the particular selected fluorescent dye and is generally in a range of 5.0-11.0, preferably in a range of 8.0-10.0.

The erythrolysis agent of the disclosure may also optionally include an appropriate proportion of an alcohol, e.g., methanol, to facilitate aggregation of the internal structures of the leukocytes.

### Leukocyte Analysis Kit

In another aspect, the disclosure further provides a kit for classifying and counting leukocytes in blood. The kit includes a leukocyte classification reagent that includes a fluorescent dye represented by general formula F: 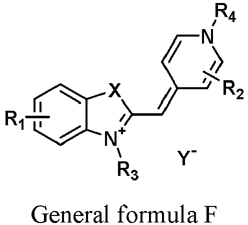
in which X is selected from the group consisting of C(CH₃)₂, O, S and Se;
R₁ and R₂ are each independently selected from the group consisting of H, C₁-C₁₈ alkyl, phenyl, OR₆ and halogen;
R₃ and R₄ are each independently selected from the group consisting of Ci-Cis alkyl, Ci-Cis-carboxyl, Ci-Cis-hydroxyl, C₁-C₁₈NR₅R₆, benzyl and substituted benzyl which is substituted with a substituent selected from the group consisting of C₁-C₁₈ alkyl, CN, COOH, NH₂, NO₂, OH, SH, C₁-C₆ alkoxy, C₁-C₆ alkylamino, C₁-C₆ amide, halogen and C₁-C₆ haloalkyl;
R₅ and R₆ are each independently selected from the group consisting of H and Ci-Cis alkyl; and
Y⁻ is an anion.

In other embodiments, the definitions of X, R₁, R₂, R₃, R₄ and Y⁻ are as described for the above fluorescent dye.

Preferably, the fluorescent dye is selected from compounds represented by chemical formula I, chemical formula II, chemical formula III, chemical formula IV, chemical formula V, chemical formula VI, chemical formula VII, chemical formula VIII, chemical formula IX, chemical formula X, chemical formula XI, chemical formula XII, chemical formula XIII, chemical formula XIV, chemical formula XV, chemical formula XVI, chemical formula XVII and chemical formula XVIII, respectively:

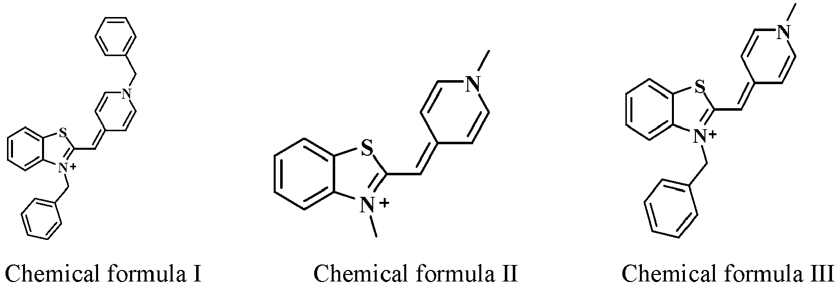

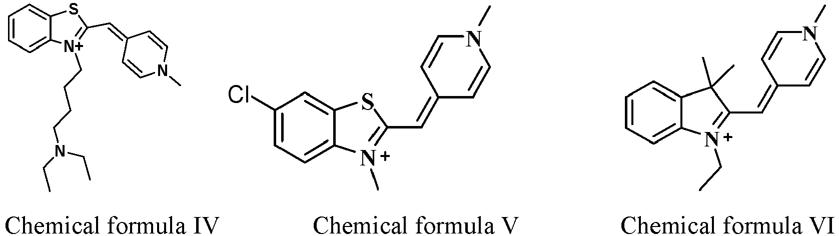

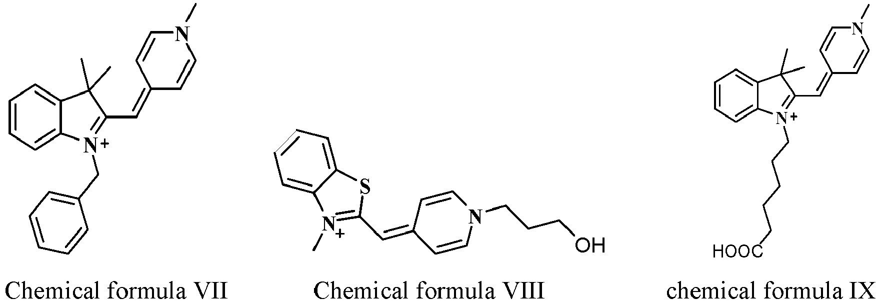

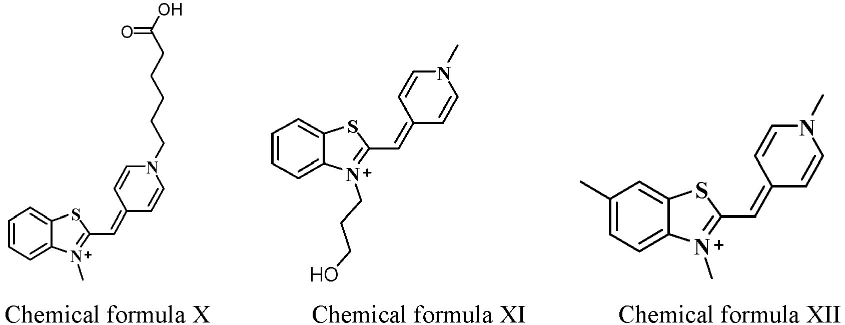

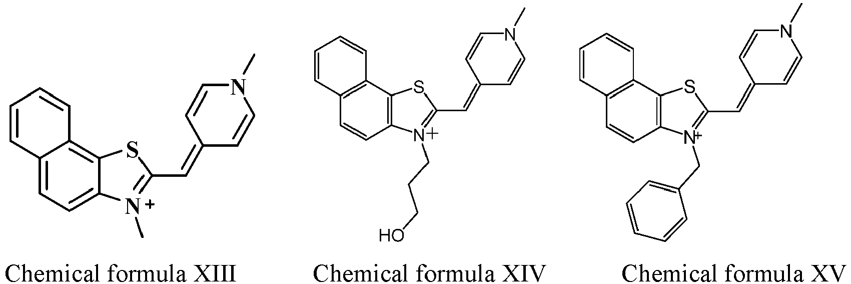

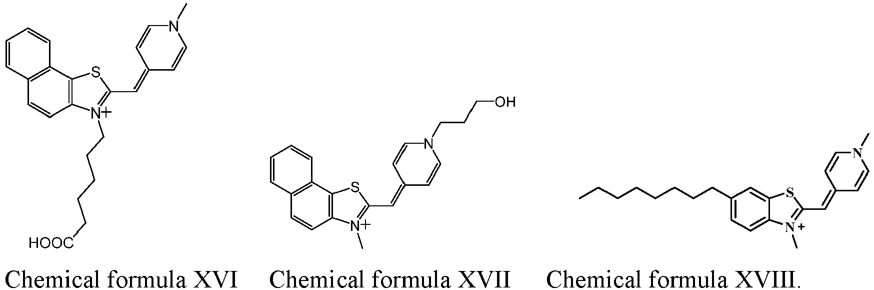

Preferably, the leukocyte classification reagent also includes the erythrolysis agent described above.

In one embodiment, the fluorescent dye is presented in the form of a stock solution and is preserved in a separate container. in which the concentration of the fluorescent dye is in the range of 0.1-1000 mg/L, preferably 20-500 mg/L, more preferably 50-200 mg/L.

In another embodiment, the fluorescent dye is formulated with the above-mentioned erythrolysis agent to obtain a mixed reagent solution.

The kit preferably includes the fluorescent dye suitable for being sealed in at least one container deposited in a partition. The kit also includes other leukocyte classification reagents required for classifying the leucocytes in blood, and the instruction of the method for analysis of leucocytes. The kit also includes a control sample or a series of control samples that can be analyzed and compared with a test sample. Each component of the kit may be packaged in a single container, and different containers, along with the instruction, are packed together in a single package. The kit is used to classify and/or count various leukocyte subsets in blood.

### Spheroidizing Reagent

The spheroidizing reagent used herein includes an amphoteric surfactant, an osmotic pressure regulator, a buffer, and optionally a cationic surfactant and/or a preservative.

The surfactant in the spheroidizing reagent causes blood cells to swell and slightly damages the cell membranes, allowing the dye to enter the cells. The concentration of the amphoteric surfactant is in the range of 100-2000 mg/L, preferably 100-500 mg/L. The amphoteric surfactant is one or more of alkyl betaine, sulfobetaine, cocoamidopropyl betaine, etc. The cationic surfactant is octyltrimethyl ammonium bromide (OTAB), decyltrimethyl ammonium bromide (DTAB), lauryltrimethyl ammonium chloride (LTAC), tetradecyltrimethylammonium bromide (CTAB), tetradecyltrimethyl ammonium chloride (CTAC), or the like. The cationic surfactant is used at a concentration of 0-50 mg/L. The osmotic pressure regulator is a commonly used salt such as sodium chloride or sodium sulfate, and the osmotic pressure is in the range of 200 mOsm/kg-380 mOsm/kg. The type of the buffer is not specifically limited, as long as it is suitable for the purposes of the disclosure, such as a carboxylate, a phosphate, a citrate, Tris-HCl, MOPS, and other organic buffers at an appropriate concentration. The suitable pH of the reagent of the disclosure varies depending on the selected particular fluorescent dye and is generally in the range of 5.0-11.0, preferably in the range of 8.0-10.0.

### Erythrocyte Analysis Kit

In another aspect, the disclosure further provides a kit for classifying and counting erythrocytes, especially reticulocytes, in blood. The kit includes an erythrocyte classification reagent that includes a fluorescent dye represented by general formula F: 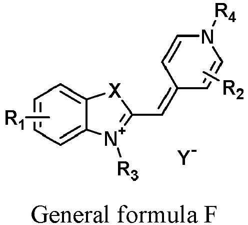
in which X is selected from the group consisting of C(CH₃)₂, O, S and Se;
R₁ and R₂ are each independently selected from the group consisting of H, C₁-C₁₈ alkyl, phenyl, OR₆ and halogen;
R₃ and R₄ are each independently selected from the group consisting of Ci-Cis alkyl, Ci-Cis-carboxyl, Ci-Cis-hydroxyl, C₁-C₁₈NR₅R₆, benzyl and substituted benzyl which is substituted with a substituent selected from the group consisting of C₁-C₁₈ alkyl, CN, COOH, NH₂, NO₂, OH, SH, C₁-C₆ alkoxy, C₁-C₆ alkylamino, C₁-C₆ amide, halogen and C₁-C₆ haloalkyl;
R₅ and R₆ are each independently selected from the group consisting of H and Ci-Cis alkyl; and
Y⁻ is an anion.

In other embodiments, the definitions of X, R₁, R₂, R₃, R₄ and Y⁻ are as described for the above fluorescent dye.

Preferably, the fluorescent dye is selected from compounds represented by chemical formula I, chemical formula II, chemical formula III, chemical formula IV, chemical formula V, chemical formula VI, chemical formula VII, chemical formula VIII, chemical formula IX, chemical formula X, chemical formula XI, chemical formula XII and chemical formula XIII, respectively: 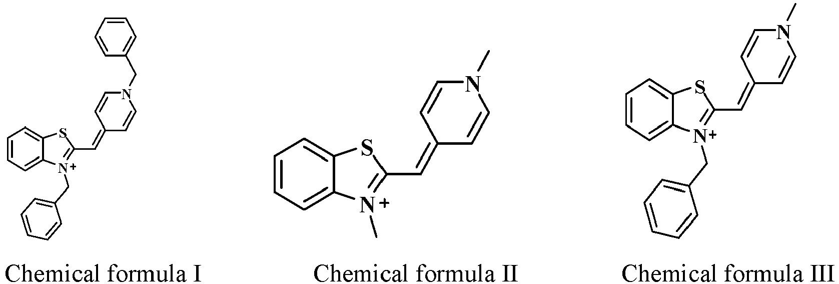 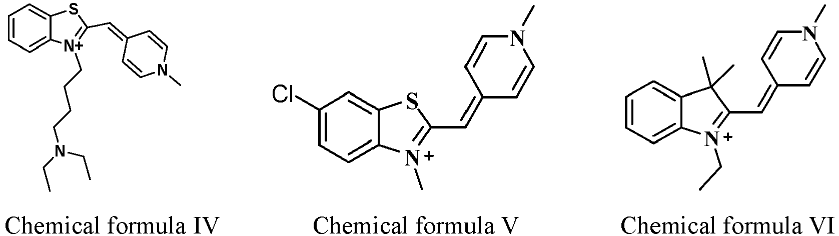 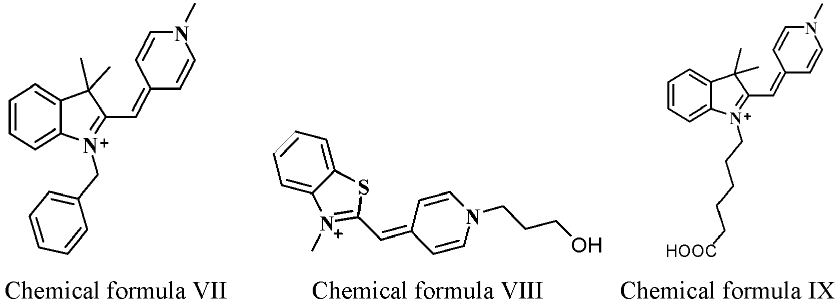 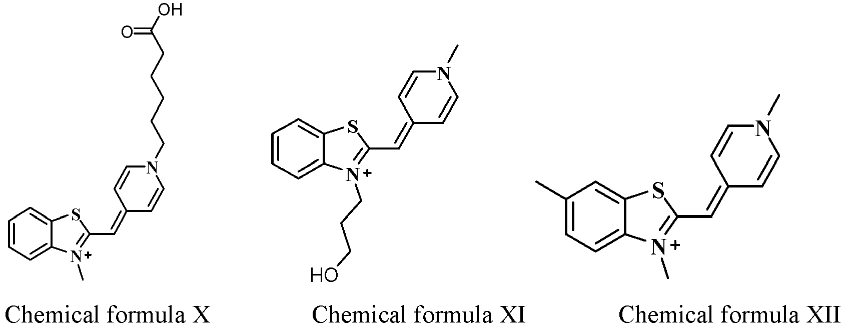 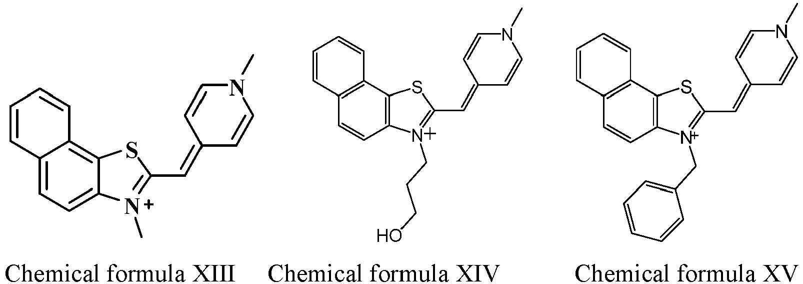 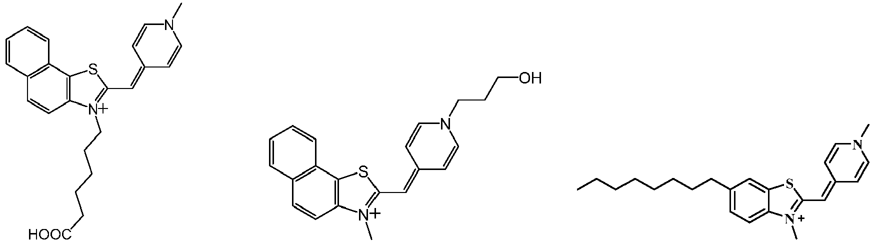 Chemical formula XVI Chemical formula XVII Chemical formula XVIII.

Preferably, the erythrocyte classification reagent also includes the spheroidizing reagent described above.

In one embodiment, the fluorescent dye is presented in the form of a stock solution and preserved in a separate container. The concentration of the fluorescent dye is in the range of 0.1-1000 mg/L, preferably 20-500 mg/L, more preferably 50-200 mg/L.

In another embodiment, the fluorescent dye is formulated with the above-mentioned spheroidizing reagent to obtain a mixed reagent solution.

The kit preferably includes a fluorescent dye suitable for being sealed in at least one container deposited in a partition. The kit also includes other erythrocyte classification reagents required for classifying the erythrocytes in blood, and the instruction of the method for analysis of erythrocytes. The kit also includes a control sample or a series of control samples that can be analyzed and compared with a test sample. Each component of the kit is packaged in a single container, and different containers, along with the instruction, are packed together in a single package. The kit is used to classify and/or count various types of erythrocytes, such as mature erythrocytes and/or reticulocytes, in blood.

### Methods of Use:

A: The disclosure further provides a method for classifying leukocytes or classifying and counting leukocytes in blood. In brief, the method of the disclosure includes: mixing a blood sample with the leukocyte classification reagent; then detecting at least one scattered light characteristic and at least one fluorescence characteristic of the sample; and classifying or classifying and counting the sample based on the scattered light characteristic and the fluorescence characteristic.

The blood sample used in the method of the disclosure is either whole blood or a blood component. The blood sample is mixed with an erythrolysis agent first, causing the erythrocytes to be lysed and various leukocyte subsets to contract to different degrees. In the step, small pores are also formed on the cell membranes of the leukocytes to be determined, allowing the fluorescent dye molecules to pass through the cell membranes.

Subsequently, a fluorescent dye stock solution is added, which enables the leukocytes to be fluorescently labeled. When mixing the blood sample with the reagent, it is necessary to ensure that the total volume of the blood sample and the reagent provides a sufficient cell concentration to pass through the measuring cell of the apparatus. The blood sample is diluted with the reagent composition of the disclosure to 1 : 10, 1 : 50 or 1 : 100, or any intermediate ratio within any of the above-mentioned ranges, so long as the dilution meets practical use requirements. Such adjustment is within the purview of a person skilled in the art. In addition, the blood sample may be mixed with the erythrolysis agent and the fluorescent dye simultaneously.

The sample mixture is incubated in an incubation cell for a period of less than 60 seconds, preferably 30 seconds, more preferably 24 seconds, at any suitable temperature, e.g., 40 °C. Both a hematology analyzer and a flow cytometer may be used to detect and analyze leukocytes. A blood sample after lysed, diluted, and stained is introduced into a flow chamber of a measurement device. Particles in the flow chamber are irradiated with the light emitted from a light source to generate optical information. A detector is configured to collect the optical information, which includes at least one type of scattered light and at least one type of fluorescence.

The term "scattered light" in the disclosure means the scattered light that can be detected by a commercially available hematology analyzer or a similar flow cytometer. Such scattered light includes, but is not limited to, side scattered light, forward low-angle scattered light (light-receiving angle of about 0-5 degrees) and forward high-angle scattered light (light-receiving angle of about 5-20 degrees). The scattered light with such angles reflects the information about the size or internal structures of the leukocytes, and therefore serving as the scattered light of the disclosure. Preferably, the scattered light is side-scattered light.

The fluorescent dye that binds to nucleic acids in cells emits fluorescence. The fluorescence characteristic is a parameter that reflects the amount of the fluorescent dye inside the cells in the blood sample. Since differences in metabolic activities of cells of various subsets lead to different contents of the nucleic acids, the fluorescence characteristics of various leukocyte subsets are different in some aspects. An excitation light with a suitable wavelength is selected depending on the particular dye used, and the emitted light of the corresponding wavelength is monitored. When the leukocytes are detected in the disclosure, laser in green or blue wavelength region, emitted by a green or blue semiconductor laser device, may be used as the light source for detection. The light source of green or blue light wavelength is not specifically limited, so long as it can emit green light (e.g., light with a wavelength of about 501-560 nm) or blue light (e.g., light with a wavelength of about 400-500 nm) close to the excitation wavelength of the selected fluorescent dye. For example, the light source may be configured to emit light with a wavelength of about 450 nm or 520 nm. Such a laser device is less expensive than other laser devices, which reduces costs of the device.

With the scattered light characteristic and the fluorescence characteristic, various leukocyte subsets are identified, and the clustered scattering points are classified and counted. The percentages of various leukocyte subsets are calculated. The scattered light reflects the granularity inside a cell. The granularity inside a cell is roughly as follows: eosinophils having two-lobulated nucleus and many crumbly particles that can be stained with acidic dyes therein; neutrophils having a (lobulated or rod-shaped) nucleus and a large quantity of particles therein; monocytes having a single large nucleus with few particles therein; and lymphocytes (LYM) having a single large nucleus but substantially having no particles therein. Therefore, the order of the intensity of the scattered light from various subsets of leukocytes under the same conditions is EO (eosinophils) > NEUT (neutrophils) > MONO (monocytes) > LYM (lymphocytes).

B: The disclosure further provides a method for classifying erythrocytes or classifying and counting erythrocytes in blood. In brief, the method of the disclosure includes: mixing a blood sample with the erythrocyte classification reagent; then detecting at least one scattered light characteristic and at least one fluorescence characteristic of the sample; and classifying or classifying and counting the cells in the sample based on the scattered light characteristic and the fluorescence characteristic.

The blood sample used in the method of the disclosure is either whole blood or a blood component. The blood sample is mixed with a spheroidizing reagent first, causing the erythrocytes and the leukocytes to swell and slightly damaging the cell membranes, such that small pores are formed on the cell membranes of the erythrocytes and the leukocytes, sufficiently allowing the fluorescent dye molecules to pass through the cell membranes.

Subsequently, a fluorescent dye stock solution is added, which enables the erythrocytes and the leukocytes to be labeled by the fluorescent dye. Since the nucleic acid contents of mature erythrocytes, reticulocytes, and leukocytes differ from each other, resulting in distinct fluorescence characteristics, and thus these three types of cells can be distinguished. When mixing the blood sample with the reagents, it is necessary to ensure that the total volume of the blood sample and the reagents of the disclosure provides a sufficient cell concentration to pass through the measuring cell of the apparatus. The blood sample may be diluted with the reagent composition of the disclosure to 1 : 10, 1 : 50 or 1 : 100, or any intermediate ratio within any of the above-mentioned ranges, so long as the dilution meets practical use requirements. Such adjustment is within the purview of a person skilled in the art. In addition, the blood sample may be mixed with the spheroidizing reagent and the fluorescent dye simultaneously.

The sample mixture is incubated in an incubation cell for a period of less than 60 seconds, preferably 30 seconds, more preferably 24 seconds. The incubation temperature is any suitable temperature, e.g., 40°C. Both a hematology analyzer and a flow cytometer can be used to detect and analyze erythrocytes. A blood sample after spheroidized and stained is introduced into the flow chamber of a measurement apparatus. Particles in the flow chamber are irradiated with the light emitted from a light source to generate optical information. A detector is configured to collect the optical information, which includes at least one type of scattered light and at least one type of fluorescence.

The term "scattered light" in the disclosure means the scattered light that can be detected by a commercially available hematology analyzer or a similar flow cytometer. Such scattered light includes, but is not limited to, side scattered light, forward low-angle scattered light (light-receiving angle of about 0-5 degrees) and forward high-angle scattered light (light-receiving angle of about 5-20 degrees). The scattered light with such angles reflects the information about the size or internal structures of the cells, and therefore serving as the scattered light of the disclosure. Preferably, the scattered light is a forward-scattered light.

When the erythrocytes are detected in the disclosure, laser in a green or blue wavelength region, emitted by a green or blue semiconductor laser device may also be used as the light source for detection.

With the scattered light characteristic and the fluorescence characteristic, mature erythrocytes, reticulocytes and platelets are identified, and the clustered scattering points are classified and counted to obtain the erythrocyte (including mature erythrocytes and reticulocytes) count, the reticulocyte count, and the platelet count. The forward scattered light reflects the information about the volume size of the cells. The platelets, which have a volume smaller than that of the erythrocytes, show a weaker forward scattered light intensity. The mature erythrocytes and the reticulocytes have different nucleic acid (RNA) contents. The reticulocytes have a relatively higher nucleic acid (RNA) content and, consequently, a relatively higher fluorescence intensity.

### Example 1

### Synthesis of Dye

An exemplary fluorescent dye is synthesized as the followings. Compound A represented by chemical formula I is prepared:

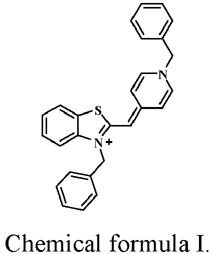

In this example, X is S, R₁ is H, R₂ is H, R₃ is benzyl, and R₄ is benzyl.

The specific method for preparing Compound A is as follows:
Step 1: 2-(methylthio)benzothiazole (the right one of Reaction I) is prepared according to the following Reaction I,

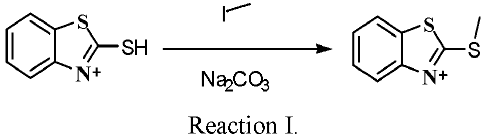

DMF (20 mL) was measured and placed into a container. 2-mercaptobenzothiazole (10 mmol, the left one of Reaction I), methane (11 mmol) and sodium carbonate (12 mmol) were added into the container. The reaction was kept on by stirring at 110°C under nitrogen protection for 8 hours and then stopped.

The resulting mixture was cooled to room temperature, and then poured into a large amount of water. The mixture was extracted three times with an appropriate amount of ethyl acetate, and the extracted organic phases were combined. The organic phases above were washed with distilled water twice, and then dried over anhydrous magnesium sulfate overnight.

Subsequently, the resulting substance after dried overnight was purified on a chromatographic column to obtain an orange-yellow solid powder, i.e., 2-(methylthio)benzothiazole (about 8.5 mmol). The yield of Reaction I is about 85%.

Step 2: 1-benzyl-4-methylpyridine quaternary ammonium salt (one right of Reaction II) is prepared according to the following Reaction II:

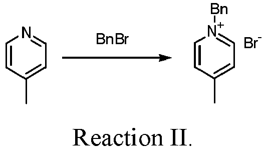

Toluene (50 mL) was measured and placed into a container. 4-methylpyridine (10 mmol, the left one of Reaction II) and benzyl bromide (12 mmol) were added into the container. The reaction was kept on under refluxing and stirring for 8 hours under nitrogen protection and then stopped.

After the reaction, the resulting mixture was suction-filtered and then the filter cake was washed three times with toluene (50 mL) to obtain a crude product.

The crude product mentioned above was purified on a chromatographic column to obtain a white powder, i.e., 1-benzyl-4-methylpyridine quaternary ammonium salt (about 7.3 mmol). The yield of Reaction II is about 73%.

Step 3: 3-benzyl-2-thione benzothiazole (the right one of Reaction III) is prepared according to the following Reaction III

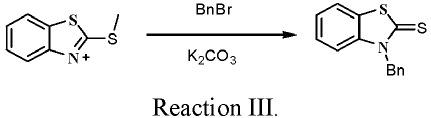

Toluene (30 mL) was measured and placed into a container. 2-(methylthio)benzothiazole (3 mmol, the left one of Reaction III) obtained in the reaction of step 1, benzyl bromide (4 mmol) and potassium carbonate (5 mmol) were added into the container. The reaction was kept on by stirring at 110°C under nitrogen protection for 8 hours.

After the reaction, the resulting mixture was suction-filtered and then the filter cake was washed three times with toluene (50 mL) to obtain a crude product.

The crude product mentioned above was purified on a chromatographic column to obtain a white solid powder, i.e., 3-benzyl-2-thione benzothiazole (about 2.1 mmol). The yield of Reaction III is about 70%.

Step 4: 3-benzyl-2-ethylthiobenzothiazole (the right one of Reaction IV) is prepared according to the following Reaction IV

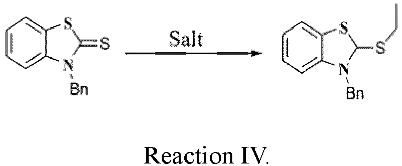

Dichloromethane (30 mL) was measured in a container. 3-benzyl-2-thione benzothiazole (2 mmol) obtained in the reaction of step 3 and salt (2 mmol) were added into the container. The reaction was kept on by refluxing and stirring for 24 hours under nitrogen protection.

The product was purified on a chromatographic column to obtain a white solid powder, i.e., 3-benzyl-2-ethylthiobenzothiazole (about 1.1 mmol) . The yield of the Reaction IV is about 55%.

Step 5: Compound A (the right one of Reaction V) is prepared according to the following Reaction V:

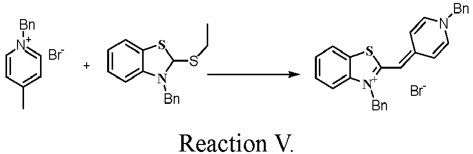

Ethanol (30 mL) was measured in a container. 1-benzyl-4-methylpyridine quaternary ammonium salt (1 mmol) obtained in the reaction of step 2 and 3-benzyl-2-ethylthiobenzothiazole (1 mmol) obtained in the reaction of step 4 were weighed and added into the container.

The reaction was kept on by refluxing and stirring for 24 hours under nitrogen protection. The product was purified on a chromatographic column to obtain an orange-yellow solid powder, i.e., Compound A (about 0.3 mmol). The yield of the Reaction V is about 30%.

Compound A was analyzed by nuclear magnetic resonance test (NMR). The result is as follows:
1H-NMR (500 MHz, DMSO, TMS): δ4.00 (s, 3H), 5.59(s, 2H), 6.39(s, 1H), 7.26-7.31(m, 4H), 7.35-7.38(t, 2H), 7.40-7.42(d, 2H), 7.45-7.48(t, 1H), 7.52-7.54(d, 1H), 7.94-7.96(d, 1H), 8.33-8.35(d, 2H).

### Example 2

Preparation of Compound J represented by chemical formula X:

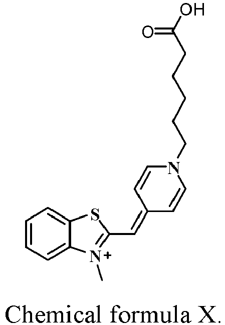

In this example, X is S, R₁ is H, R₂ is H, R₃ is carboxypentyl, and R₄ is methyl.

The specific method for preparing Compound J is as follows.

Step 1: To a 50 mL round-bottomed flask was added ethyl acetate (10 mL). Then 4-methylpyridine (5.37 mmol) and 6-bromohexanoic acid (10.7 mmol) were added to the round-bottomed flask, and the mixture was reacted under refluxing at 80°C for 8 h. The resulting mixture was recrystallized with ethyl acetate and suction-filtered. The filtrate was sufficiently washed with ethyl acetate during suction filtration. The resulting filtrate was dissolved in methanol, and then dried by evaporation. A yellow oily liquid, i.e., 1-(5-carboxypentyl)-4-methylpyridine, was obtained with a crude yield of about 91.07%.

Step 2: To a 50 mL round-bottomed flask was added anhydrous tetrahydrofuran (10 mL). Subsequently, 2-mercaptobenzothiazole (11.84 mmol) was added to the round-bottomed flask. Iodomethane (14.21 mmol) was then slowly added dropwise to the round-bottomed flask under stirring, and the mixture was reacted overnight. Afterwards, a small amount of petroleum ether was added to the resulting mixture, which was then left to stand for precipitation. After filtration, the filtrate was washed with a large amount of petroleum ether. Finally, the filtrate was dried to obtain a white solid, i.e., 2-(methylthio)benzothiazole. The crude yield is about 98%.

Step 3: To a 50 mL round-bottomed flask was added toluene (10 mL). Then 2-(methylthio)benzothiazole (10 mmol) was added into the round-bottomed flask. Afterwards, under nitrogen protection, iodomethane (12 mmol) was slowly added dropwise into the round-bottomed flask and the mixture was reacted at 110°C under refluxing for 24 h. The resulting reaction mixture was cooled to room temperature, and then filtered. The filtrate was washed with dichloromethane. Finally, the filtrate was dried to obtain a yellowish solid powder, i.e., 3-methyl-2-(methylthio)benzothiazole. The crude yield is about 30%.

Step 4: To a 25 mL round-bottomed flask, dichloromethane (5 mL) was added. Then, 3-methyl-2-(methylthio)benzothiazole (0.500 mmol) and 1-(5-carboxypentyl)-4-methylpyridine (0.509 mmol) were added into the round-bottomed flask. After the mixture was stirred at room temperature in dark for 10 min, triethylamine (756 µL) was slowly added dropwise, and then the resulting mixture was reacted at room temperature in dark for about 21 h. Afterwards, the resulting reaction mixture was dried by evaporation, and the residue was separated on a neutral silica column with a mixed solvent of dichloromethane and methanol as an eluent. The separated yellow fraction was collected and dried by evaporation to finally obtain a brownish viscous substance, i.e., Compound J. The crude yield is 50%.

Compound J was analyzed by nuclear magnetic resonance test (NMR). The result is as follows:
¹H-NMR (400MHz, DMSO): δ 8.36 (s, 1H), 7.90 (d, J=7.6Hz, 1H), 7.58(d, J=8.0Hz, 1H), 7.51(t, J=7.4Hz, 1H), 7.40 (s, 1H), 7.30 (t, J=7.1Hz, 1H), 6.25 (s, 1H), 4.21 (s, 1H), 3.72 (s, 3H), 2.10 (s, 1H), 1.82 (s, 1H), 1.53 (s, 1H), 1.28 (s, 1H).

### Example 3:

### Formulation of Reagent

A reagent system consisting of the following components is formulated.
A: Fluorescent dye stock solution:

| | |
|---|---|
| Fluorescent dye (with structure of chemical formula I) | 50 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1 L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Cocoamidopropyl betaine | 100 mg |
| Water | 1 L |
| pH | 8.5 |

### 1) Determination of leukocytes in a blood sample and results:

The process of detecting a sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

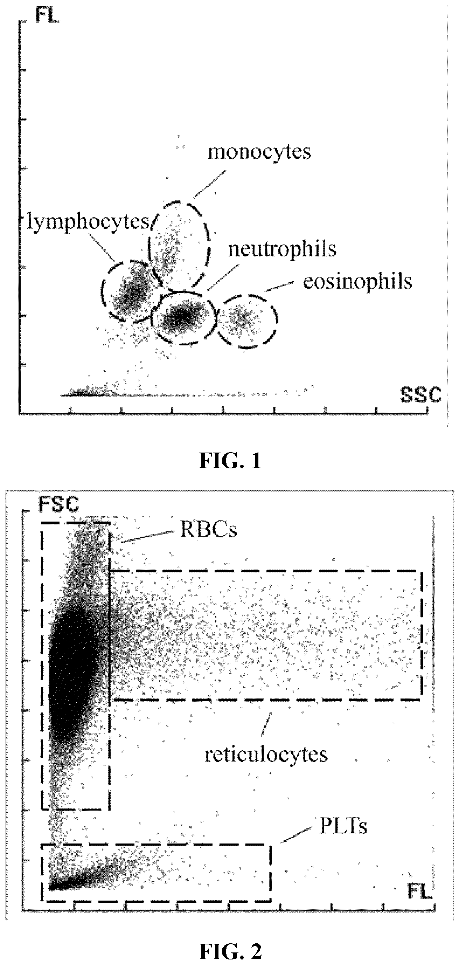

Specifically, 1 ml of the erythrolysis agent was mixed uniformly with 20 microliters of anti-coagulated blood and then immediately added with 20 microliters of the fluorescent dye stock solution, the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 1) are shown in FIG. 1, which shows that the disclosure can achieve the classification and identification of four leukocyte subsets. The four clusters of particles marked in FIG. 1 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results:

The process of detecting a sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution, the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 1) are shown in FIG. 2, which shows that the disclosure can achieve the classification and identification of erythrocytes and platelets. The three clusters of particles marked in FIG. 2 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 1) was detected on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 3 | |
|---|---|---|---|---|
| Percentage of lymphocytes (%) | | 37.8 | | 37.2 |
| Percentage of monocytes (%) | | 8.1 | | 8.3 |
| Percentage of neutrophils (%) | | 52.9 | | 52.7 |
| Percentage of eosinophils (%) | | 1.2 | | 1.3 |
| Percentage of reticulocytes (%) | | 0.8 | | 1.1 |

It can be seen from the table that the testing results of the fluorescent dye represented by chemical formula I on the leukocyte subsets and the reticulocytes are nearly consistent with the testing results obtained using the BC-6800 analyzer.

### Example 4:

### Formulation of Reagent

A reagent system consisting of the following components is formulated.
A: Fluorescent dye stock solution:

| | |
|---|---|
| Fluorescent dye (with structure of chemical formula II) | 50 mg |
| Ethylene glycol | 950 ml |
| Methanol | 50 ml |

B: Erythrolysis agent solution

| | |
|---|---|
| Lauryltrimethyl ammonium chloride | 0.5 g |
| TRIS | 18 g |
| HCl | 20 ml |
| Water | 1L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1L |
| pH | 8.5 |

### 1) Determination of leukocytes in a blood sample and results:

The process of detecting a sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

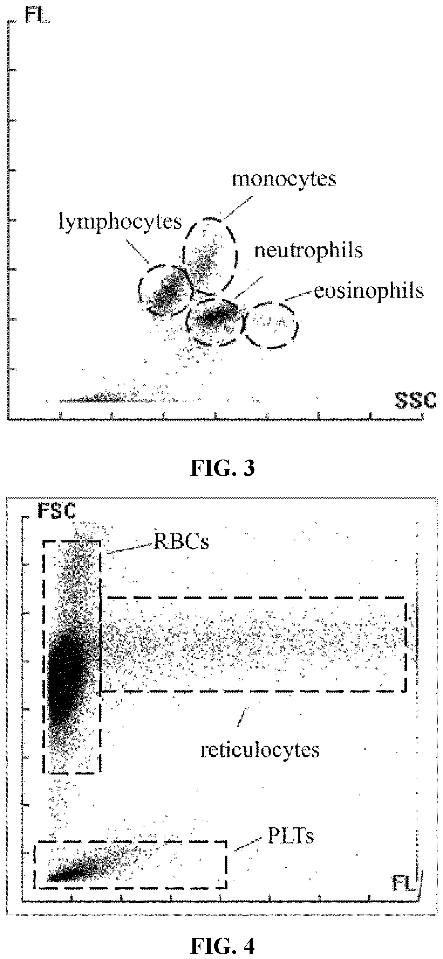

Specifically, 1 ml of the erythrolysis agent was mixed uniformly with 20 microliters of anti-coagulated blood and then immediately added with 20 microliters of the fluorescent dye stock solution, the mixture was then uniformly mixed and incubated in an incubation cell at 40°C for 30 seconds, so that a test sample is prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 2) are shown in FIG. 3, which shows that the disclosure can achieve the classification and identification of four leukocyte subsets. The four clusters of particles marked in FIG. 3 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results:

The process of detecting sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 2) are shown in FIG. 4, which shows that the disclosure can achieve the classification and identification of erythrocytes and platelets. The three clusters of particles marked in FIG. 4 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 2) was detected on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 4 | |
|---|---|---|---|---|
| Percentage of lymphocytes (%) | | 44.1 | | 43.6 |
| Percentage of monocytes (%) | | 4.2 | | 4.3 |
| Percentage of neutrophils (%) | | 51.1 | | 53.3 |
| Percentage of eosinophils (%) | | 0.6 | | 0.7 |
| Percentage of reticulocytes (%) | | 0.9 | | 1.1 |

It can be seen from the table that the testing results of the fluorescent dye represented by chemical formula II on the leukocyte subsets and the reticulocytes are nearly consistent with the testing results obtained using the BC-6800 analyzer.

### Example 5:

### Formulation of Reagent

A reagent system consisting of the following components is formulated.
A: Fluorescent dye stock solution:

| | |
|---|---|
| Fluorescent dye (with structure of chemical formula III) | 50 mg |
| Ethylene glycol | 950 ml |
| Methanol | 50 ml |

B: Erythrolysis agent solution

| | |
|---|---|
| Lauryltrimethyl ammonium chloride | 0.5 g |
| TRIS | 18 g |
| HCl | 20 ml |
| Water | 1L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1L |
| pH | 8.5 |

### 1) Determination of leukocytes in a blood sample and results:

The process of detecting a sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

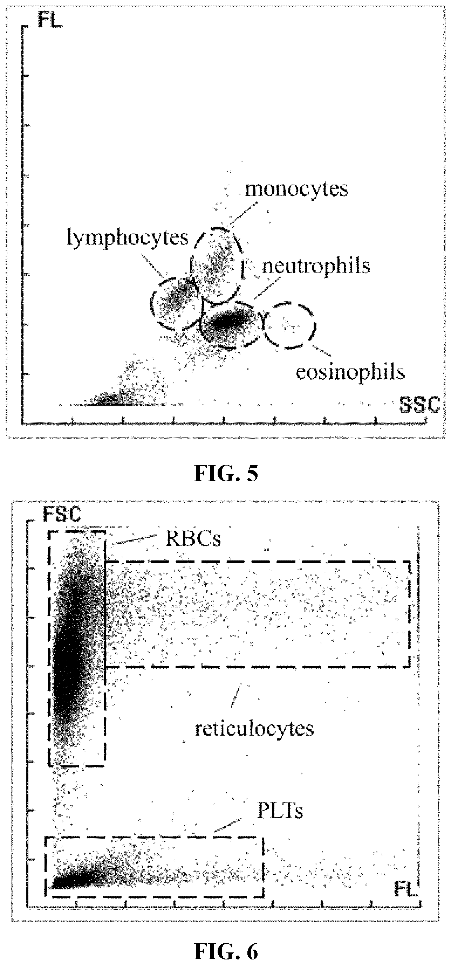

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 3) are shown in FIG. 5, which shows that the disclosure can achieve the classification and identification of four leukocyte subsets. The four clusters of particles marked in FIG. 5 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results:

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 3) are shown in FIG. 6, which shows that the disclosure can achieve the classification and identification of erythrocytes and platelets. The three clusters of particles marked in FIG. 6 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 3) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | Testing results of reagent formulated in example 5 |
|---|---|---|
| Percentage of lymphocytes (%) | 33.9 | 33.2 |
| Percentage of monocytes (%) | 8.7 | 8.3 |
| Percentage of neutrophils (%) | 57 | 58 |
| Percentage of eosinophils (%) | 0.4 | 0.5 |
| Percentage of reticulocytes (%) | 0.3 | 0.4 |

It can be seen from the table that the testing results of the fluorescent dye represented by chemical formula III on the leukocyte subsets and the reticulocytes are nearly consistent with the testing results obtained using the BC-6800 analyzer.

### Example 6:

### Formulation of Reagent

A reagent system consisting of the following components is formulated.
A: Fluorescent dye stock solution:

| | |
|---|---|
| Fluorescent dye (with structure of chemical formula IV) | 100 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1L |
| pH | 8.5 |

### 1) Determination of leukocytes in a blood sample and results:

The process of detecting a sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

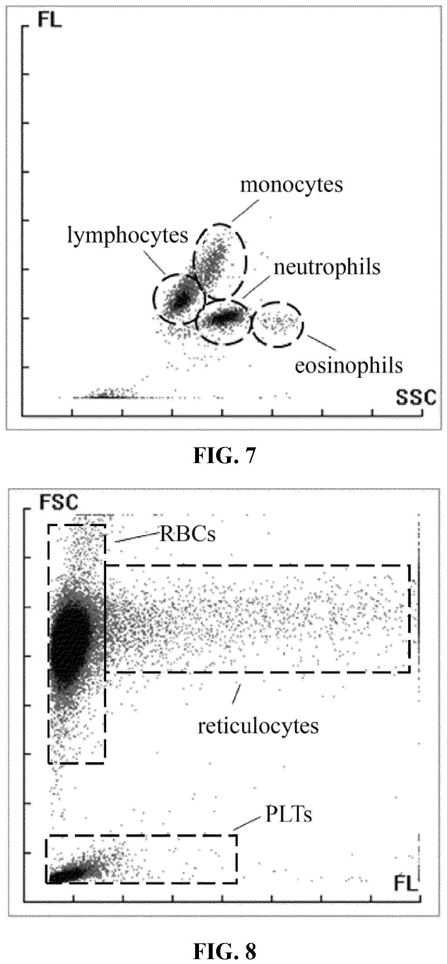

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 4) are shown in FIG. 7, which shows that the disclosure can achieve the classification and identification of four leukocyte subsets. The four clusters of particles marked in FIG. 7 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results:

The process of detecting the sample testing process was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution was added immediately to the mixture, which was then uniformly mixed and incubated in an incubation cell at 40°C for 30 seconds. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 4) are shown in FIG. 8, which shows that the disclosure can achieve the classification and identification of erythrocytes and platelets. The three clusters of particles marked in FIG. 8 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 4) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 6 | |
|---|---|---|---|---|
| Percentage of lymphocytes (%) | | 40.2 | | 39.8 |
| Percentage of monocytes (%) | | 12.3 | | 11.9 |
| Percentage of neutrophils (%) | | 46.8 | | 47.8 |
| Percentage of eosinophils (%) | | 0.6 | | 0.5 |
| Percentage of reticulocytes (%) | | 0.5 | | 0.6 |

It can be seen from the table that the testing results of the fluorescent dye represented by chemical formula IV on the leukocyte subsets and the reticulocytes are nearly consistent with the testing results obtained using the BC-6800 analyzer.

### Example 7:

### Formulation of Reagent

A reagent system consisting of the following components is formulated.
A: Fluorescent dye stock solution:

| | |
|---|---|
| Fluorescent dye (with structure of chemical formula V) | 500 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1L |
| pH | 8.5 |

### 1) Determination of leukocytes in blood sample and results:

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

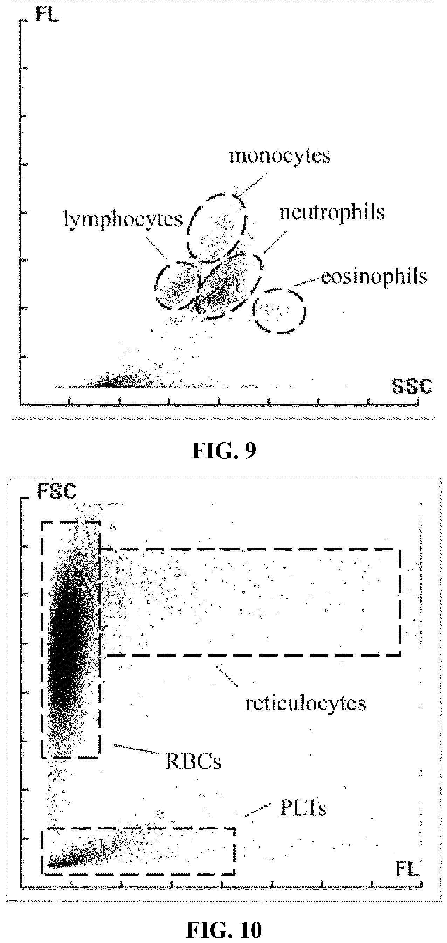

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 5) are shown in FIG. 9, which shows that the disclosure can achieve the classification and identification of four leukocyte subsets. The four clusters of particles marked in FIG. 9 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 5) are shown in FIG. 10, which shows that the disclosure can achieve the classification and identification of erythrocytes and platelets. The three clusters of particles marked in FIG. 10 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 5) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 7 | |
|---|---|---|---|---|
| Percentage of lymphocytes (%) | | 28.6 | | 29.2 |
| Percentage of monocytes (%) | | 10.5 | | 10.7 |
| Percentage of neutrophils (%) | | 59.8 | | 58.8 |
| Percentage of eosinophils (%) | | 1.1 | | 1.3 |
| Percentage of reticulocytes (%) | | 0.3 | | 0.3 |

It can be seen from the table that the testing results of the fluorescent dye represented by chemical formula V on the leukocyte subsets and the reticulocytes are nearly consistent with the testing results obtained using the BC-6800 analyzer.

### Example 8:

### Formulation of Reagent

A reagent system consisting of the following components is formulated.
A: Fluorescent dye stock solution:

| | |
|---|---|
| Fluorescent dye (with structure of chemical formula VI) | 50 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1L |
| pH | 8.5 |

### 1) Determination of leukocytes in blood sample and results:

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

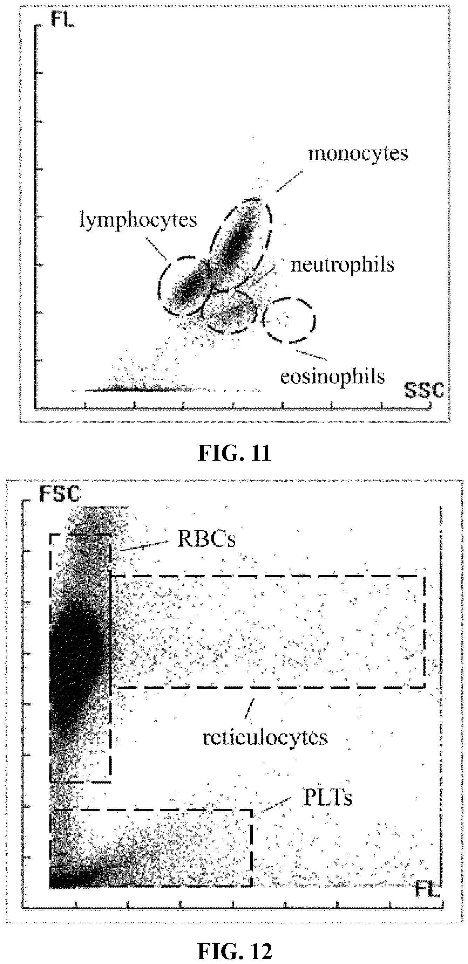

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 6) are shown in FIG. 11, which shows that the disclosure can achieve the classification and identification of four leukocyte subsets. The four clusters of particles marked in FIG. 11 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results:

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 6) are shown in FIG. 12, which shows that the disclosure can achieve the classification and identification of erythrocytes and platelets. The three clusters of particles marked in FIG. 12 are erythrocytes (namely, mature erythrocytes), reticulocytes (RET) and platelets, respectively. It demonstrates that the erythrocyte detection reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 6) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 8 | |
|---|---|---|---|---|
| Percentage of lymphocytes (%) | | 37.7 | | 37.2 |
| Percentage of monocytes (%) | | 40.6 | | 41.3 |
| Percentage of neutrophils (%) | | 21.2 | | 20.9 |
| Percentage of eosinophils (%) | | 0.5 | | 0.6 |
| Percentage of reticulocytes (%) | | 0.4 | | 0.4 |

It can be seen from the table that the testing results of the fluorescent dye represented by chemical formula VI on the leukocyte subsets and the reticulocytes are nearly consistent with the testing results obtained using the BC-6800 analyzer.

### Example 9:

### Formulation of Reagent

A reagent system consisting of the following components is formulated.
A: Fluorescent dye stock solution:

| | |
|---|---|
| Fluorescent dye (with structure of chemical formula VII) | 250 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1 L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 200 mg |
| Water | 1 L |
| pH | 8.5 |

### 1) Determination of leukocytes in blood sample and results:

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

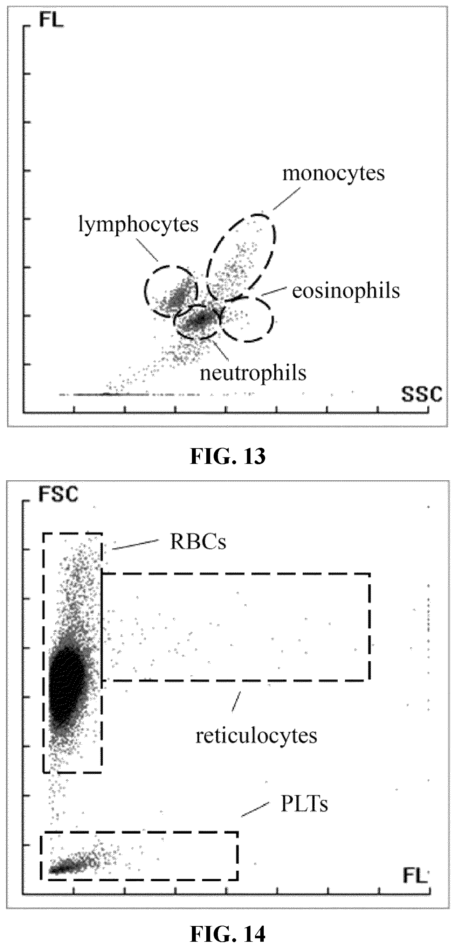

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatter diagram of leukocytes. The testing results of the blood sample (blood sample No. 7) are shown in FIG. 13, which shows that the disclosure can achieve the classification and identification of four leukocyte subsets. The four clusters of particles marked in FIG. 13 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results:

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 7) are shown in FIG. 14, which shows that the disclosure can achieve the classification and identification of erythrocytes and platelets. The three clusters of particles marked in FIG. 14 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 7) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 9 | |
|---|---|---|---|---|
| Percentage of lymphocytes (%) | | 36.3 | | 37.2 |
| Percentage of monocytes (%) | | 10.2 | | 11.0 |
| Percentage of neutrophils (%) | | 52.4 | | 50.5 |
| Percentage of eosinophils (%) | | 1.1 | | 1.3 |
| Percentage of reticulocytes (%) | | 0.1 | | 0.1 |

It can be seen from the table that the testing results of the fluorescent dye represented by chemical formula VII on the leukocyte subsets and the reticulocytes are nearly consistent with the testing results obtained using the BC-6800 analyzer.

### Example 10:

### Formulation of Reagent

A reagent system consisting of the following components is formulated.
A: Fluorescent dye stock solution:

| | |
|---|---|
| Fluorescent dye (with structure of chemical formula VIII) | 50 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1 L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1 L |
| pH | 8.5 |

### 1) Determination of leukocytes in blood sample and results:

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

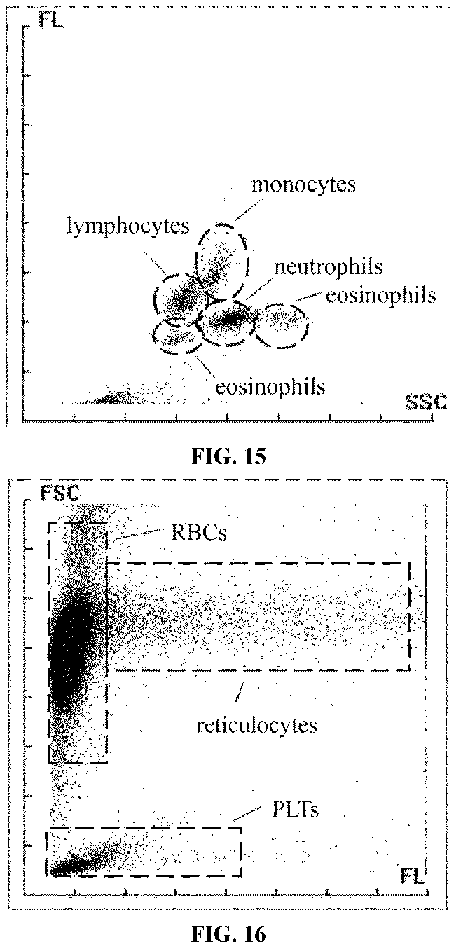

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 8) are shown in FIG. 15, which shows that the disclosure can achieve the classification and identification of five leukocyte subsets. The five clusters of particles marked in FIG. 15 are lymphocytes, monocytes, neutrophils, eosinophils, and basophils, respectively. It demonstrates that the leukocyte classification reagent of the disclosure can achieve the effective classification of different leukocyte subsets.

### 2) Determination of erythrocytes in blood sample and results

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 8) are shown in FIG. 16, which shows that the disclosure can achieve the classification and identification of erythrocytes and platelets. The three clusters of particles marked in FIG. 16 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 8) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | Testing results of reagent formulated in example 10 |
|---|---|---|
| Percentage of lymphocytes (%) | 33.6 | 33.2 |
| Percentage of monocytes (%) | 10.2 | 10.8 |
| Percentage of neutrophils (%) | 54.1 | 54.0 |
| Percentage of eosinophils (%) | 1.1 | 1.2 |
| Percentage of basophils (%) | 1.0 | 0.8 |
| Percentage of reticulocytes (%) | 1.2 | 1.1 |

It can be seen from the table that the testing results of the fluorescent dye represented by chemical formula VIII on the leukocyte subsets and the reticulocytes are nearly consistent with the testing results obtained using the BC-6800 analyzer.

### Example 11:

### Formulation of Reagent

A reagent system consisting of the following components is formulated.
A: Fluorescent dye stock solution:

| | |
|---|---|
| Fluorescent dye (with structure of chemical formula IX) | 50 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Cocoamidopropyl betaine | 100 mg |
| Water | 1 L |
| pH | 8.5 |

### 1) Determination of leukocytes in blood sample and results

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

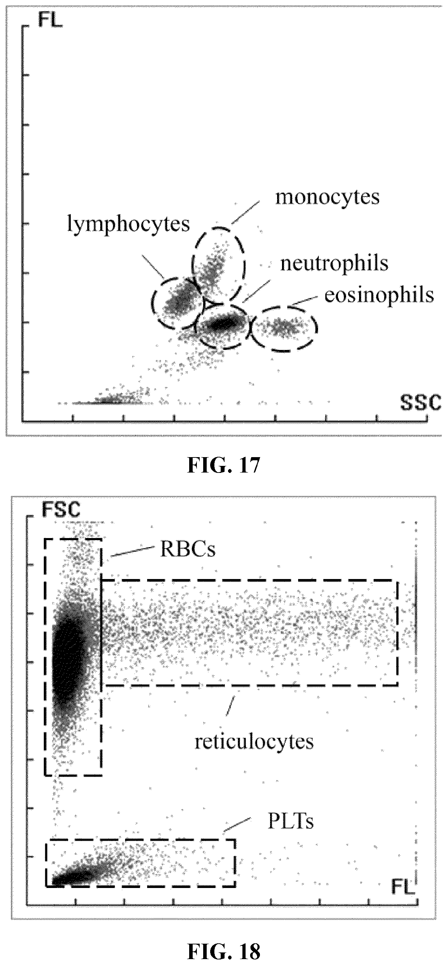

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 9) are shown in FIG. 17, which shows that the disclosure can achieve the classification and identification of four leukocyte subsets. The four clusters of particles marked in FIG. 17 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 9) are shown in FIG. 18, which shows that the disclosure can achieve the classification and identification of erythrocytes and platelets. The three clusters of particles marked in FIG. 18 are erythrocytes (namely, mature erythrocytes), reticulocytes (RET) and platelets, respectively. It demonstrates that the erythrocyte detection reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 9) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 11 | |
|---|---|---|---|---|
| Percentage of lymphocytes (%) | | 39.4 | | 39.2 |
| Percentage of monocytes (%) | | 8.8 | | 8.5 |
| Percentage of neutrophils (%) | | 49.7 | | 50.0 |
| Percentage of eosinophils (%) | | 2.1 | | 2.3 |
| Percentage of reticulocytes (%) | | 0.8 | | 0.7 |

It can be seen from the table that the testing results of the fluorescent dye represented by chemical formula IX on the leukocyte subsets and the reticulocytes are nearly consistent with the testing results obtained using the BC-6800 analyzer.

### Example 12:

### Formulation of Reagent

A reagent system consisting of the following components is formulated.
A: Fluorescent dye stock solution:

| | |
|---|---|
| Fluorescent dye (with structure of chemical formula X) | 50 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1 L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1 L |
| pH | 8.5 |

### 1) Determination of leukocytes in blood sample and results

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

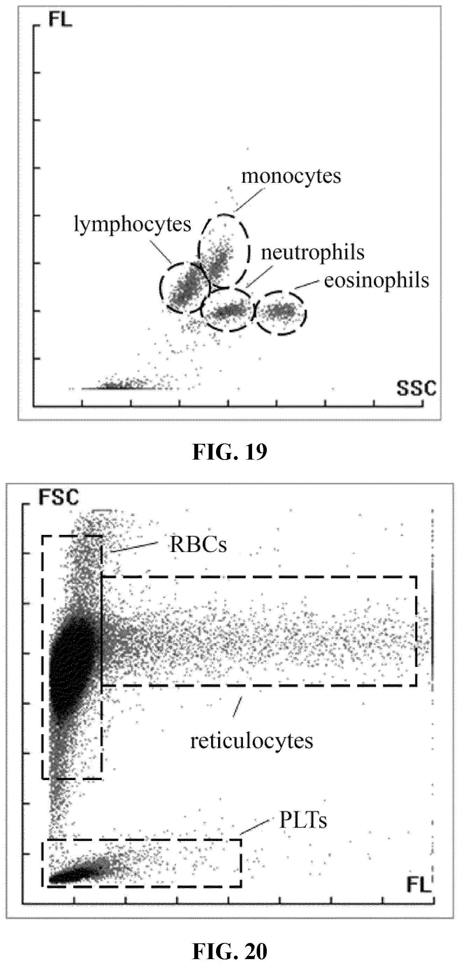

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 10) are shown in FIG. 19, which shows that the disclosure can achieve the classification and identification of four leukocyte subsets. The four clusters of particles marked in FIG. 19 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results

The process of detecting the sample was automatically performed in a hematology analyzer independently developed by Mindray. The sampling quantity was set to 4 microliters, the sample feeding quantity of the spheroidizing reagent solution C was 1 ml, the sample feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 10) are shown in FIG. 20, which shows that the disclosure can achieve the classification and identification of erythrocytes and platelets. The three clusters of particles marked in FIG. 20 are erythrocytes (namely, mature erythrocytes), reticulocytes (RET) and platelets, respectively. It demonstrates that the erythrocyte detection reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 10) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 12 | |
|---|---|---|---|---|
| Percentage of lymphocytes (%) | | 39.3 | | 39.9 |
| Percentage of monocytes (%) | | 12.4 | | 12.1 |
| Percentage of neutrophils (%) | | 42.2 | | 41.8 |
| Percentage of eosinophils (%) | | 6.1 | | 6.2 |
| Percentage of reticulocytes (%) | | 1.9 | | 2.1 |

It can be seen from the table that the testing results of the fluorescent dye represented by chemical formula X on the leukocyte subsets and the reticulocytes are nearly consistent with the testing results obtained using the BC-6800 analyzer.

### Example 13:

### Formulation of Reagent

A reagent system consisting of the following components is formulated.
A: Fluorescent dye stock solution:

| | |
|---|---|
| Fluorescent dye (with structure of chemical formula XI) | 50 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1 L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1 L |
| pH | 8.5 |

### 1) Determination of leukocytes in blood sample and results

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

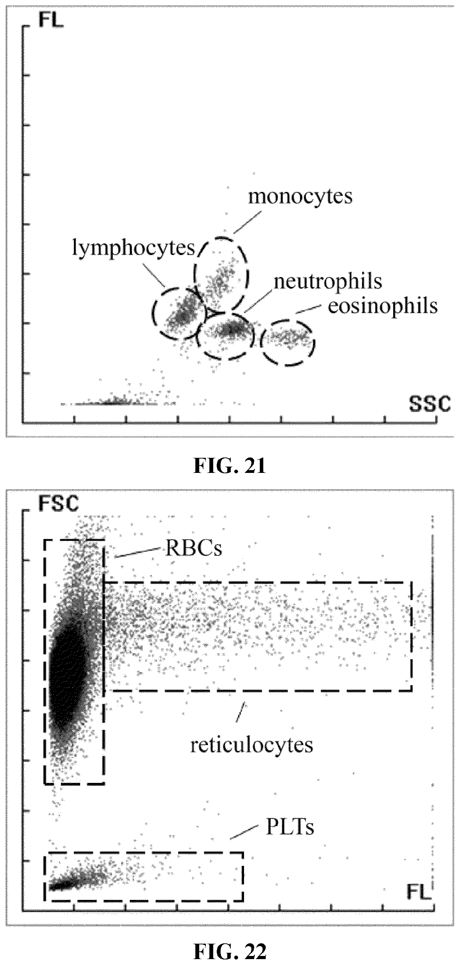

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 11) are shown in FIG. 21, which shows that the disclosure can achieve the classification and identification of four leukocyte subsets. The four clusters of particles marked in FIG. 21 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 11) are shown in FIG. 22, which shows that the disclosure can achieve the classification and identification of erythrocytes and platelets. The three clusters of particles marked in FIG. 22 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 11) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 13 | |
|---|---|---|---|---|
| Percentage of lymphocytes (%) | | 40.1 | | 39.8 |
| Percentage of monocytes (%) | | 9.3 | | 10.0 |
| Percentage of neutrophils (%) | | 48.4 | | 48.1 |
| Percentage of eosinophils (%) | | 2.2 | | 2.1 |
| Percentage of reticulocytes (%) | | 1.1 | | 1.0 |

It can be seen from the table that the testing results of the fluorescent dye represented by chemical formula XI on the leukocyte subsets and the reticulocytes are nearly consistent with the testing results obtained using the BC-6800 analyzer.

### Example 14:

### Formulation of Reagent

A reagent system consisting of the following components is formulated.
A: Fluorescent dye stock solution:

| | |
|---|---|
| Fluorescent dye (with structure of chemical formula XII) | 1000 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1 L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Cocoamidopropyl betaine | 100 mg |
| Water | 1 L |
| pH | 8.5 |

### 1) Determination of leukocytes in blood sample and results

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

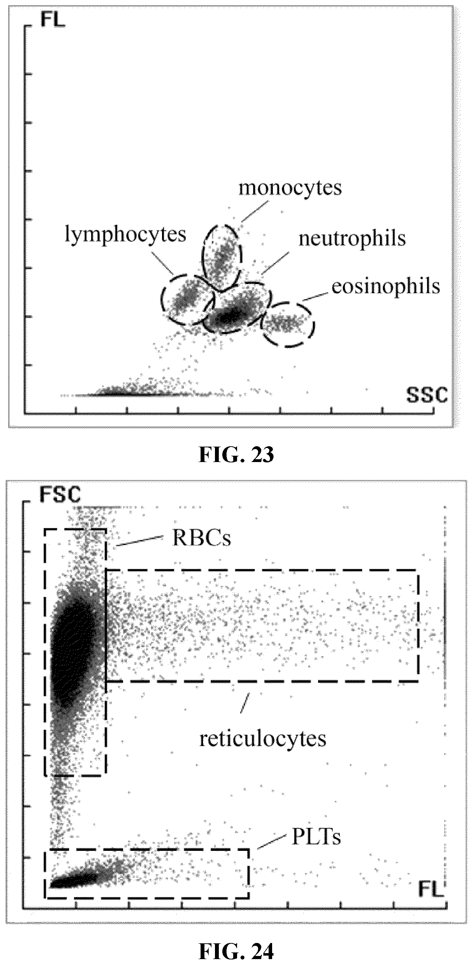

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 12) are shown in FIG. 23, which shows that the disclosure can achieve the classification and identification of four leukocyte subsets. The four clusters of particles marked in FIG. 23 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 12) are shown in FIG. 24, which shows that the disclosure can achieve the classification and identification of erythrocytes and platelets. The three clusters of particles marked in FIG. 24 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 12) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 14 | |
|---|---|---|---|---|
| Percentage of lymphocytes (%) | | 33.1 | | 33.3 |
| Percentage of monocytes (%) | | 13.2 | | 13.8 |
| Percentage of neutrophils (%) | | 52.2 | | 51.5 |
| Percentage of eosinophils (%) | | 1.5 | | 1.4 |
| Percentage of reticulocytes (%) | | 0.8 | | 0.9 |

It can be seen from the table that the testing results of the fluorescent dye represented by chemical formula XII on the leukocyte subsets and the reticulocytes are nearly consistent with the testing results obtained using the BC-6800 analyzer.

### Example 15:

### Formulation of Reagent

A reagent system consisting of the following components is formulated.
A: Fluorescent dye stock solution:

| | |
|---|---|
| Fluorescent dye (with structure of chemical formula XIII) | 50 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1L |
| pH | 8.5 |

### 1) Determination of leukocytes in blood sample and results

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

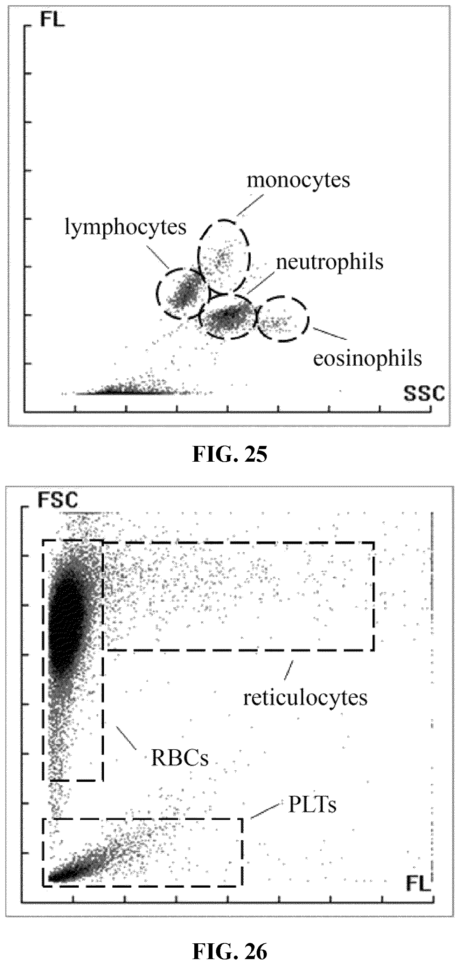

Specifically, 1 ml of the erythrolysis agent was mixed uniformly with 20 microliters of anti-coagulated blood and immediately added with 20 microliters of the fluorescent dye stock solution; the mixture, was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 13) are shown in FIG. 25, which shows that the disclosure can achieve the classification and identification of four leukocyte subsets. The four clusters of particles marked in FIG. 25 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 13) are shown in FIG. 26, which shows that the disclosure can achieve the classification and identification of erythrocytes and platelets. The three clusters of particles marked in FIG. 26 are erythrocytes (namely, mature erythrocytes), reticulocytes (RET) and platelets, respectively. It demonstrates that the erythrocyte detection reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 13) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 15 | |
|---|---|---|---|---|
| Percentage of lymphocytes (%) | | 40.6 | | 40.5 |
| Percentage of monocytes (%) | | 6.5 | | 6.7 |
| Percentage of neutrophils (%) | | 51.7 | | 51.4 |
| Percentage of eosinophils (%) | | 1.2 | | 1.4 |
| Percentage of reticulocytes (%) | | 0.7 | | 0.7 |

It can be seen from the table that the testing results of the fluorescent dye represented by chemical formula XIII on the leukocyte subsets and the reticulocytes are nearly consistent with the testing results obtained using the BC-6800 analyzer.

### Example 16:

### Formulation of Reagent

A reagent system consisting of the following components is formulated.
A: Fluorescent dye stock solution:

| | |
|---|---|
| Fluorescent dye (with structure of chemical formula XVIII) | 50 mg |
| Ethylene glycol | 950 ml |
| Methanol | 50 ml |

B: Erythrolysis agent solution

| | |
|---|---|
| Tetradecyltrimethyl ammonium chloride | 0.5 g |
| TRIS | 18 g |
| HCl | 20 ml |
| Water | 1L |
| Brij 35 | 1.5 g |
| pH | 7.0 |

C: Spheroidizing reagent solution

| | |
|---|---|
| Sodium dihydrogen phosphate monohydrate | 53 mg |
| Disodium hydrogen phosphate heptahydrate | 160 mg |
| Alkyl betaine | 100 mg |
| Water | 1L |
| pH | 8.5 |

### 1) Determination of leukocytes in blood sample and results

The process of detecting the sample was automatically performed in the improved BC-6000 hematology analyzer independently developed by Mindray. The sampling quantity was set to 20 microliters, the feeding quantity of the erythrolysis agent solution B was 1 ml, the feeding quantity of the fluorescent dye stock solution A was 20 microliters, and the blood sample was anti-coagulated blood.

Specifically, 1 ml of the erythrolysis agent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The side scattered light intensity and the fluorescence intensity of the test sample were detected to obtain a scatterplot of leukocytes. The testing results of the blood sample (blood sample No. 13) are shown in FIG. 25, which shows that the disclosure can achieve the classification and identification of four leukocyte subsets. The four clusters of particles marked in FIG. 25 are lymphocytes, monocytes, neutrophils, and eosinophils, respectively. It demonstrates that the leukocyte classification reagent of the disclosure can achieve the effective classification of different leukocyte subsets.

### 2) Determination of erythrocytes in blood sample and results

The process of detecting the sample was automatically performed in a hematology analyzer independently developed by Mindray. The sampling quantity was set to 4 microliters, the feeding quantity of the spheroidizing reagent solution C was 1 ml, the feeding quantity of the fluorescent dye stock solution A was 20 microliters, and the blood sample was anti-coagulated blood.

Specifically, 1 ml of the spheroidizing reagent was mixed uniformly with 20 microliters of anti-coagulated blood and immediately added with 20 microliters of the fluorescent dye stock solution; the mixture was then uniformly mixed and incubated in an incubation cell at 40°C for 30 seconds, so that the test sample was prepared. The hematology analyzer was provided with a blue laser device with an excitation wavelength of 450 nm. The forward scattered light intensity and the fluorescence intensity of the test sample were detected to obtain a scatterplot including erythrocytes. The testing results of the blood sample (blood sample No. 13) are shown in FIG. 26, which shows that the disclosure can achieve the classification and identification of erythrocytes and platelets. The three clusters of particles marked in FIG. 26 are erythrocytes (namely, mature erythrocytes), reticulocytes (RET) and platelets, respectively. It demonstrates that the erythrocyte classification reagent of the disclosure can achieve the classification and identification of erythrocytes and platelets, especially RETs.

### 3) Determination of erythrocytes and leukocytes in the same blood sample by Mindray BC-6800 analyzer and results.

The same blood sample (blood sample No. 13) was tested on the Mindray BC-6800 analyzer according to the CDR mode and using the supporting reagents of the BC-6800 analyzer. Relevant parameters of leukocytes and erythrocytes from individual detections are shown in the following table:

| Parameters | Testing results of BC-6800 | | Testing results of reagent formulated in example 15 | |
|---|---|---|---|---|
| Percentage of lymphocytes (%) | | 42.6 | | 42.5 |
| Percentage of monocytes (%) | | 5.5 | | 5.7 |
| Percentage of neutrophils (%) | | 50.7 | | 50.4 |
| Percentage of eosinophils (%) | | 1.1 | | 1.3 |
| Percentage of reticulocytes (%) | | 0.8 | | 0.8 |

It can be seen from the table that the testing results of the fluorescent dye represented by chemical formula XVIII on the leukocyte subsets and the reticulocytes are nearly consistent with the testing results obtained using the BC-6800 analyzer.

The disclosure has been described through the above-mentioned examples, but it should be understood that the above-mentioned examples are only for illustrative purposes and are not intended to limit the disclosure to the scope of the examples described. In addition, those skilled in the art could understand that the disclosure is not limited to the above examples. According to the teachings of the disclosure, more modifications and variations can be made, which fall within the protection scope claimed by the disclosure. The protection scope of the disclosure is defined by the appended claims and their equivalent scopes.

## Claims

1. A leukocyte classification reagent, comprising a fluorescent dye represented by general formula F: 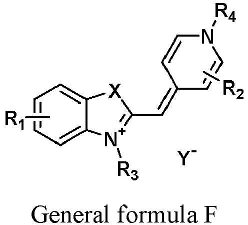
wherein X is selected from a group consisting of C(CH₃)₂, O, S and Se;
R₁ and R₂ are each independently selected from a group consisting of H, Ci-Cis alkyl, phenyl, OR₆ and halogen;
R₃ and R₄ are each independently selected from a group consisting of C₁-C₁₈ alkyl, C₁-C₁₈-carboxyl, C₁-C₁₈-hydroxyl, C₁-C₁₈NR₅R₆, benzyl and substituted benzyl which is substituted with a substituent selected from a group consisting of C₁-C₁₈ alkyl, CN, COOH, NH₂, NO₂, OH, SH, C₁-C₆ alkoxy, C₁-C₆ alkylamino, C₁-C₆ amide, halogen and C₁-C₆ haloalkyl;
R₅ and R₆ are each independently selected from a group consisting of H and Ci-Cis alkyl; and
Y⁻ is an anion.

2. The leukocyte classification reagent of claim 1, wherein X is selected from a group consisting of C(CH₃)₂ and S.

3. The leukocyte classification reagent of claim 1, wherein R₁ and R₂ are each independently selected from a group consisting of H, C₁-C₁₂ alkyl, phenyl, OR₆ and halogen;
preferably, R₁ and R₂ are each independently selected from a group consisting of H, C₁-C₆ alkyl, phenyl, OR₆ and halogen; and
more preferably, R₁ is selected from a group consisting of H, C₁-C₆ alkyl, phenyl and halogen, and R₂ is H.

4. The leukocyte classification reagent of claim 1, wherein R₃ and R₄ are each independently selected from a group consisting of C₁-C₁₂ alkyl, C₁-C₁₂-carboxyl, C₁-C₁₂-hydroxyl, C₁-C₁₂NR₅R₆, benzyl and substituted benzyl which is substituted with a substituent selected from a group consisting of C₁-C₁₂ alkyl, CN, COOH, NH₂, NO₂, OH, SH, C₁-C₆ alkoxy, C₁-C₆ alkylamino, C₁-C₆ amide, halogen and C₁-C₆ haloalkyl;
preferably, R₃ and R₄ are each independently selected from group consisting of C₁-C₆ alkyl, C₁-C₆-carboxyl, C₁-C₆-hydroxyl, C₁-C₆NR₅R₆, benzyl and substituted benzyl which is substituted with a substituent selected from a group consisting of C₁-C₆ alkyl, CN, COOH, NH₂, NO₂, OH, SH, C₁-C₆ alkoxy, C₁-C₆ alkylamino, C₁-C₆ amide, halogen and C₁-C₆ haloalkyl;
more preferably, R₃ is selected from a group consisting of C₁-C₆ alkyl, C₁-C₆-hydroxyl, C₁-C₆-carboxyl, C₁-C₆NR₅R₆ and benzyl; and R₄ is selected from a group consisting of C₁-C₆ alkyl, C₁-C₆-hydroxyl, C₁-C₆-carboxyl and benzyl.

5. The leukocyte classification reagent of claim 1, wherein R₅ and R₆ are each independently selected from a group consisting of H and C₁-C₁₂ alkyl;
preferably, R₅ and R₆ are each independently selected from a group consisting of H and C₁-C₆ alkyl; and
more preferably, R₅ and R₆ are each independently C₁-C₆ alkyl.

6. The leukocyte classification reagent of claim 1, wherein Y⁻ is selected from a group consisting of an anion of halogen, ClO₄⁻, PF₆⁻, BF₄⁻, CH₃COO⁻ or OTs⁻.

7. The leukocyte classification reagent of claim 1, wherein the fluorescent dye is represented by at least one of chemical formula I, chemical formula II, chemical formula III, chemical formula IV, chemical formula V, chemical formula VI, chemical formula VII, chemical formula VIII, chemical formula IX, chemical formula X, chemical formula XI, chemical formula XII, chemical formula XIII, chemical formula XIV, chemical formula XV, chemical formula XVI, chemical formula XVII and chemical formula XVIII:

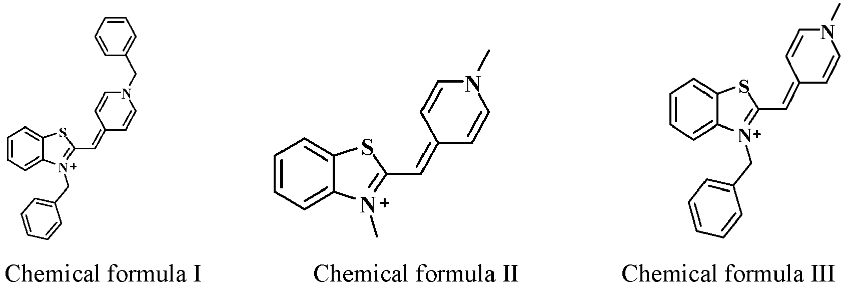

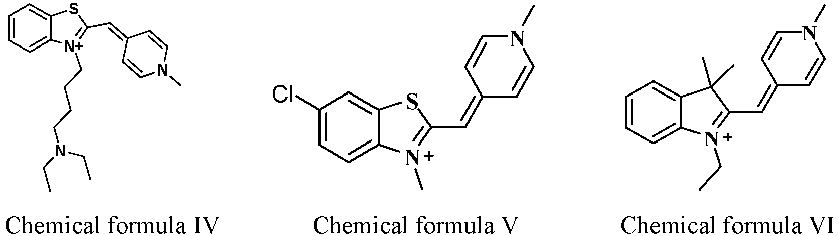

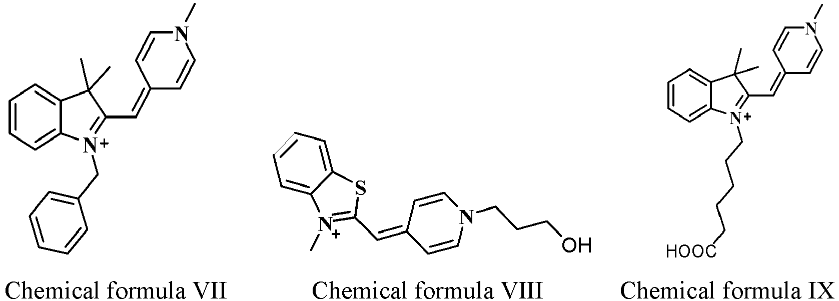

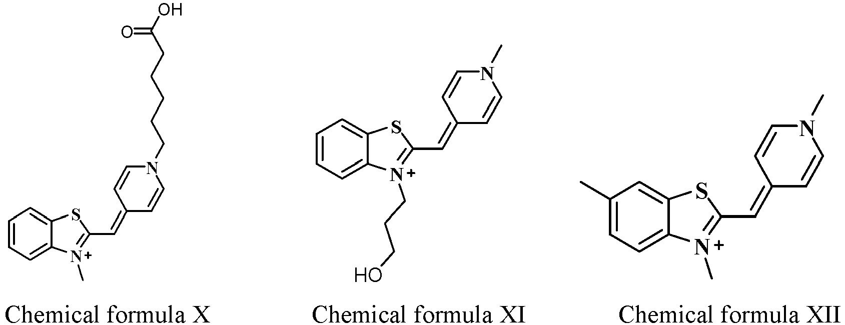

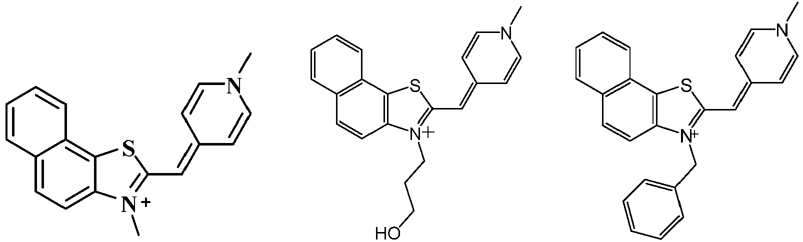

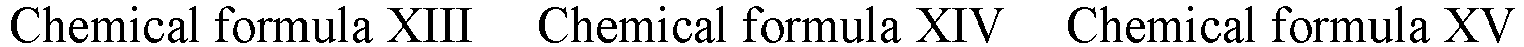

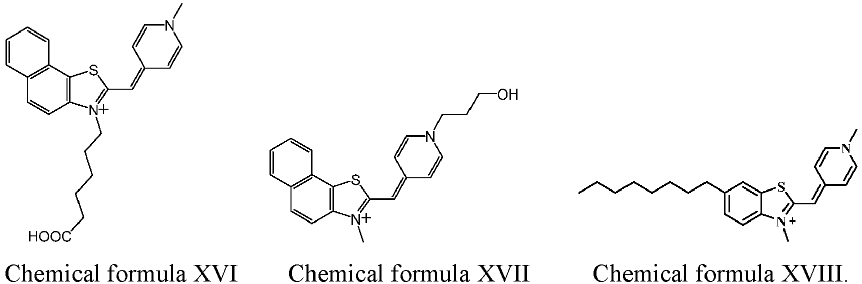

8. The leukocyte classification reagent of any one of claims 1-7, further comprising an erythrolysis agent.

9. The leukocyte classification reagent of any one of claims 1-8, wherein a concentration of the fluorescent dye is in a range of 0.1-1000 mg/L, preferably 20-500 mg/L, more preferably 50-200 mg/L.

10. The leukocyte classification reagent of claim 8, wherein the erythrolysis agent comprises a cationic surfactant, a non-ionic surfactant, an anionic surfactant, or any combination thereof; and a buffer that maintains pH of a tested system in a range of 5-11.

11. A leukocyte analysis kit, comprising the leukocyte classification reagent of any one of claims 1-10.

12. A method for analyzing leukocytes, comprising:
mixing a blood sample with a fluorescent dye represented by general formula F and an erythrolysis agent to form a sample to be tested: 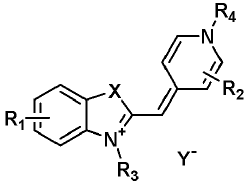
wherein X, R₁, R₂, R₃, R₄ and Y⁻ are as defined in any one of claims 1-6;
detecting at least one scattered light characteristic and at least one fluorescence characteristic of the sample to be tested by irradiating particles in the sample to be tested with light; and
classifying leukocytes, or classifying and counting leukocytes based on the scattered light characteristic and the fluorescence characteristic.

13. The method of claim 12, wherein the fluorescent dye represented by general formula F has a structure of at least one of chemical formula I, chemical formula II, chemical formula III, chemical formula IV, chemical formula V, chemical formula VI, chemical formula VII, chemical formula VIII, chemical formula IX, chemical formula X, chemical formula XI, chemical formula XII, chemical formula XIII, chemical formula XIV, chemical formula XV, chemical formula XVI, chemical formula XVII and chemical formula XVIII:

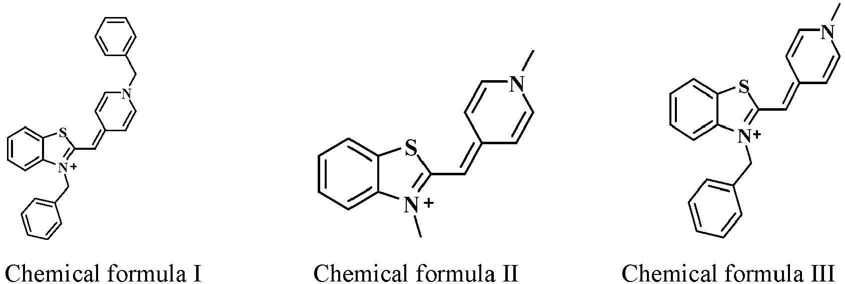

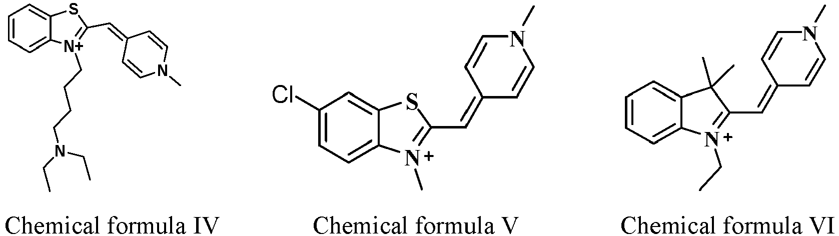

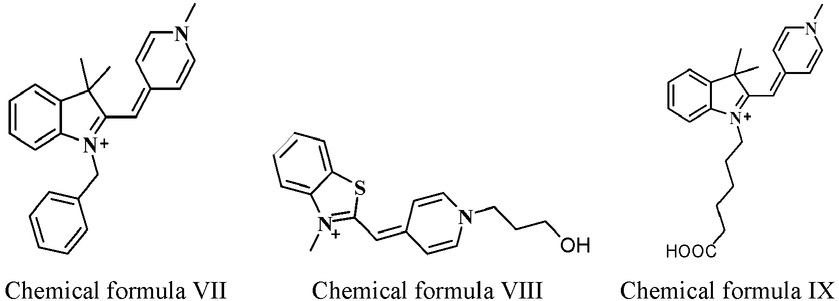

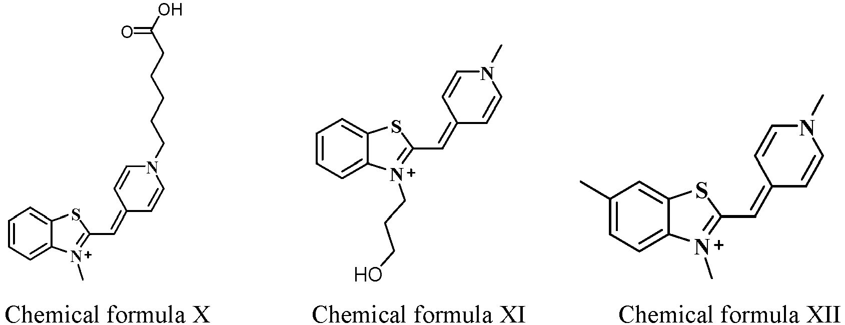

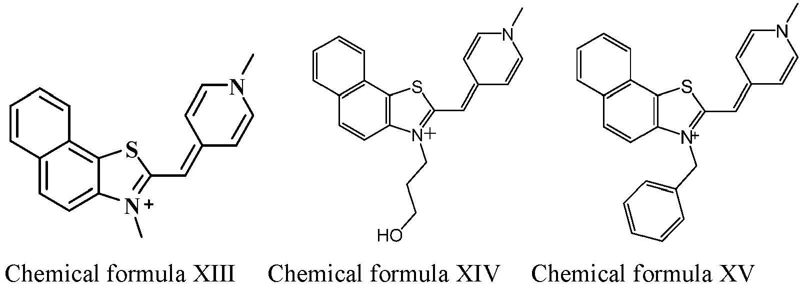

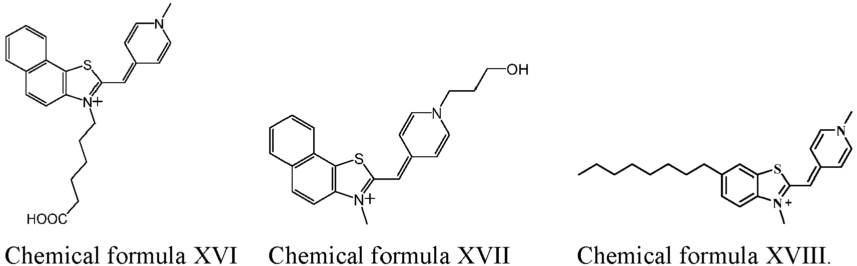

14. The method of any one of claims 12-13, wherein the light for irradiating the particles in the sample to be tested is: blue light, green light or light having an emission wavelength of 400-560 nm.

15. An erythrocyte classification reagent, comprising a fluorescent dye represented by general formula F: 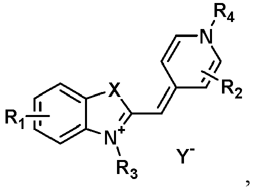 wherein X, R₁, R₂, R₃, R₄ and Y⁻ are as defined in any one of claims 1-6.

16. The erythrocyte classification reagent of claim 15, wherein the fluorescent dye is represented by at least one of chemical formula I, chemical formula II, chemical formula III, chemical formula IV, chemical formula V, chemical formula VI, chemical formula VII, chemical formula VIII, chemical formula IX, chemical formula X, chemical formula XI, chemical formula XII, chemical formula XIII, chemical formula XIV, chemical formula XV, chemical formula XVI, chemical formula XVII and chemical formula XVIII:

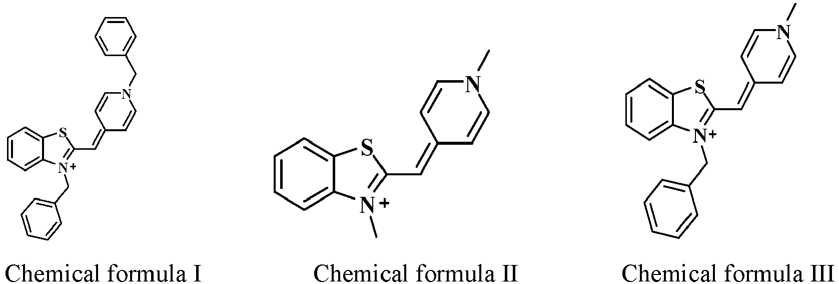

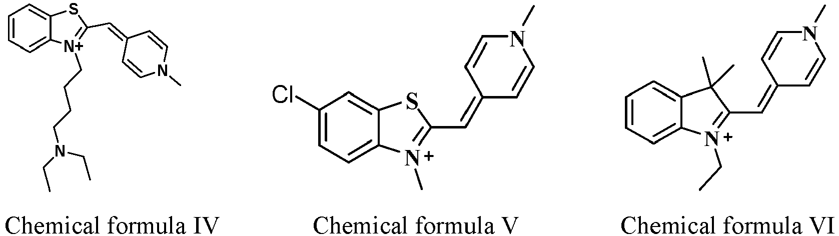

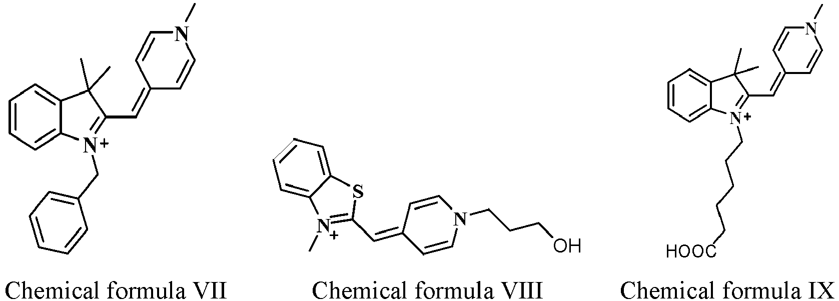

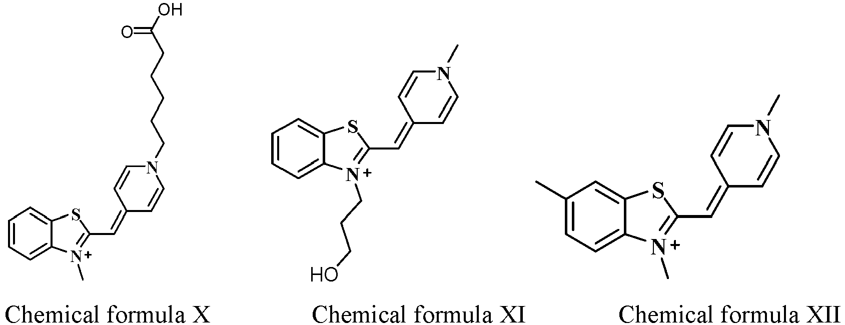

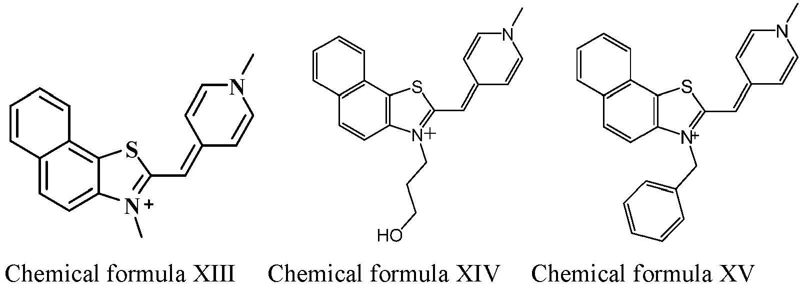

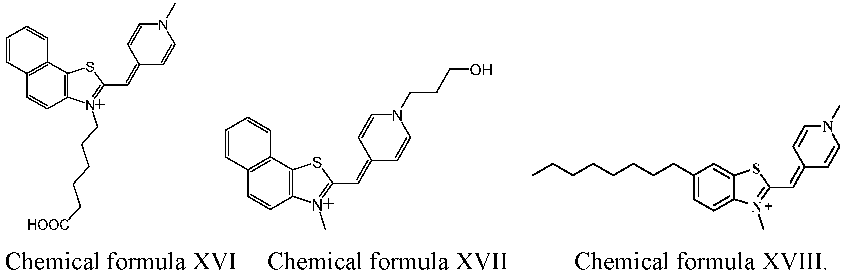

17. The erythrocyte classification reagent of claim 15, further comprising a spheroidizing reagent.

18. The erythrocyte classification reagent of claim 17, wherein the spheroidizing reagent comprises an amphoteric surfactant, an osmotic pressure regulator and a buffer.

19. The erythrocyte classification reagent of any one of claims 15-18, wherein a concentration of the fluorescent dye is in a range of 0.1-1000 mg/L, preferably 20-500 mg/L, more preferably 50-200 mg/L.

20. An erythrocyte analysis kit, comprising an erythrocyte classification reagent of any one of claims 15-19.

21. An erythrocyte analysis method, comprising:
mixing a blood sample, a fluorescent dye represented by general formula F and a spheroidizing reagent to form a sample to be tested: 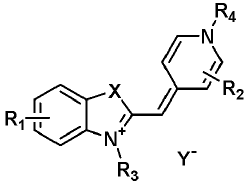
wherein X, R₁, R₂, R₃, R₄ and Y⁻ are as defined in any one of claims 1-6;
detecting at least one scattered light characteristic and at least one fluorescence characteristic of the sample to be tested by irradiating particles in the sample to be tested with light; and
classifying erythrocytes, or classifying and counting erythrocytes based on the scattered light characteristic and the fluorescence characteristic.

22. The analysis method of claim 21, wherein classifying erythrocytes comprises: classifying the erythrocytes into mature erythrocytes and/or reticulocytes; and counting erythrocytes comprises: counting the mature erythrocytes and/or the reticulocytes.

23. The analysis method of claim 22, further comprising: obtaining a count of platelets based on the scattered light characteristic and the fluorescence characteristic.

24. The analysis method of any one of claims 21-23, wherein the fluorescent dye represented by general formula F has a structure of at least one of chemical formula I, chemical formula II, chemical formula III, chemical formula IV, chemical formula V, chemical formula VI, chemical formula VII, chemical formula VIII, chemical formula IX, chemical formula X, chemical formula XI, chemical formula XII, chemical formula XIII, chemical formula XIV, chemical formula XV, chemical formula XVI, chemical formula XVII and chemical formula XVIII:

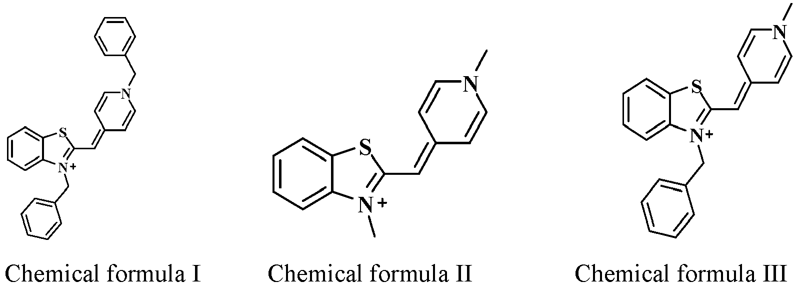

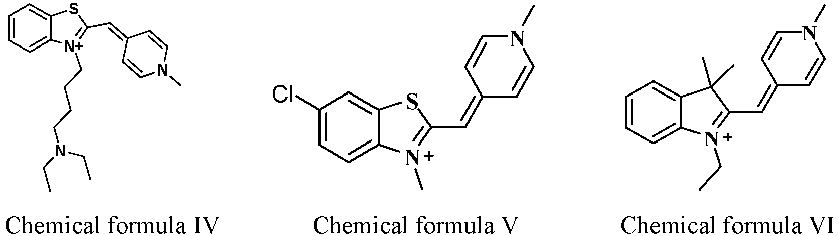

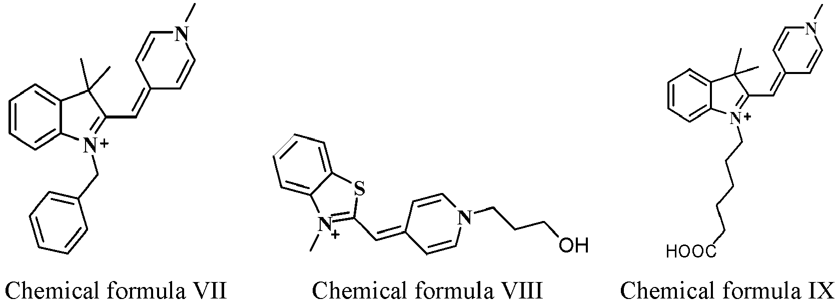

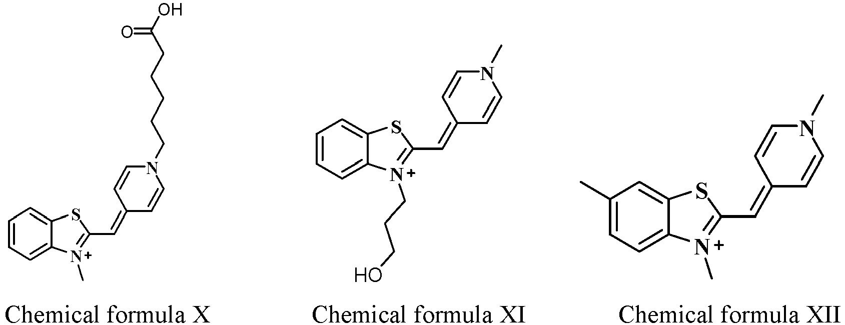

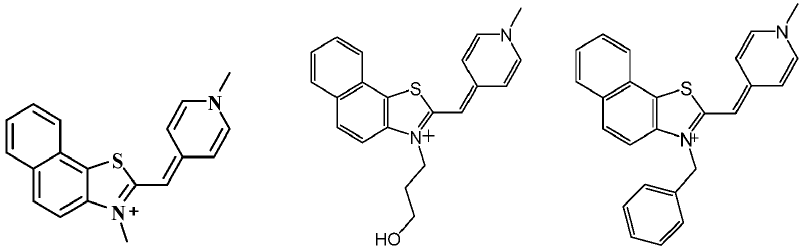

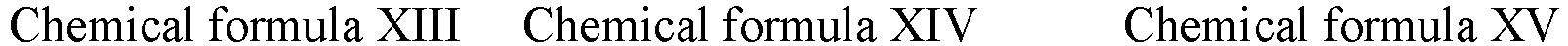

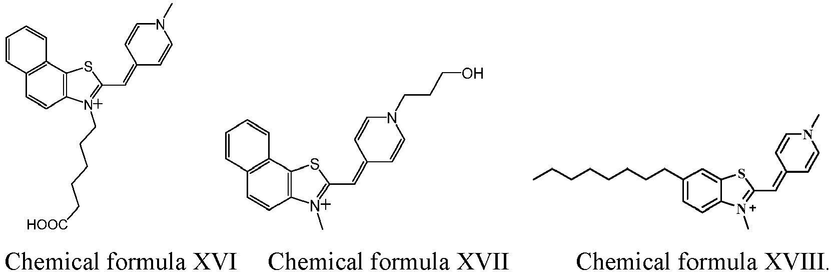

25. The method of any one of claims 21-24, wherein the light for irradiating the particles in the sample to be tested is: blue light, green light or light having an emission wavelength of 400-560 nm.
